# EUROPEAN PATENT SPECIFICATION

(11) **EP 4 420 654 B1**
(45) Date of publication and mention of the grant of the patent: **18.02.2026**
(21) Application number: 21962857.5
(22) Date of filing: 04.11.2021
(51) Int. Cl.: C07K 5/117, A61K 47/55, A61K 47/54, A61K 8/64, A61K 8/49, A61Q 19/00, A61Q 19/02, A61K 38/07, A61K 47/64, A61K 38/00

(54) **TETRAPEPTIDE DERIVATIVE, COSMETIC COMPOSITION OR PHARMACEUTICAL COMPOSITION AND USE THEREOF**
TETRAPEPTIDDERIVAT, KOSMETISCHE ZUSAMMENSETZUNG ODER PHARMAZEUTISCHE ZUSAMMENSETZUNG UND VERWENDUNG DAVON
DÉRIVÉ DE TÉTRAPEPTIDE, COMPOSITION COSMÉTIQUE OU COMPOSITION PHARMACEUTIQUE ET LEUR UTILISATION

(43) Date of publication of application: 28.08.2024
(73) Proprietor: Shenzhen Winkey Technology Co., Ltd, Shenzhen, Guangdong 518000 (CN)
(72) Inventor: DING, Wenfeng, Shenzhen, Guangdong 518100 (CN); PENG, Yan, Shenzhen, Guangdong 518100 (CN); CHEN, Xue, Shenzhen, Guangdong 518100 (CN); ZHAO, Wenhao, Shenzhen, Guangdong 518100 (CN); SUN, Xinlin, Shenzhen, Guangdong 518100 (CN); HUANG, Chunqing, Shenzhen, Guangdong 518100 (CN); GUAN, Fuyi, Shenzhen, Guangdong 518100 (CN); XIAO, Yu, Shenzhen, Guangdong 518100 (CN)
(74) Representative: Bayramoglu et al.
(86) International application number: PCT/CN2021/128561
(87) International publication number: WO 2023/077339

(56) References cited:
- WO-A2-2009/068351
- CN-A- 102 573 776
- CN-A- 103 957 914
- CN-A- 109 311 938
- KR-A- 20170 011 283
- SINGH BIRENDRA KUMAR, PARK SEOK HOON, LEE HYANG-BOK, GOO YOUNG-AAE, KIM HYOUNG SHIK, CHO SEUNG HEE, LEE JEONG HUN, AHN GHE WHAN, K: "Kojic Acid Peptide: A New Compound with Anti-Tyrosinase Potential", ANNALS OF DERMATOLOGY, KOREAN DERMATOLOGICAL ASSOCIATION, SEOUL, KR, vol. 28, no. 5, 1 January 2016 (2016-01-01), KR , pages 555 - 561, XP093063422, ISSN: 1013-9087, DOI: 10.5021/ad.2016.28.5.555

## Description

### TECHNICAL FIELD

The present application relates to a peptide derivative, a cosmetic composition or pharmaceutical composition comprising the peptide derivative and use thereof in the treatment or care of the skin and mucosa.

### BACKGROUND

The skin is exposed to the external environment and is easily affected by various physical, chemical and pathogenic microorganism stimulations, which will cause cell damage to the skin tissue and trigger the inflammatory reaction. The inflammatory reaction will not only increase skin permeability, causing collagenase to release and degrading collagen and elastin, so that the skin is present in a sagging and aging state; moreover, the produced inflammatory factors such as interleukin IL-1α, IL-6 and tumor necrosis factor TNF-α will stimulate the migration, proliferation and differentiation of melanocytes, induce keratinocytes to produce proopiomelanocortin (POMC) and α-melanocyte-stimulating hormone (α-MSH), and enhance the activity of tyrosinase so that more melanins are generated in the skin, and the skin surface becomes darker or spots appear locally. Compositions and methods for skin whitening are known. Document KR20170011283A discloses compositions comprising peptide-coupled kojic acid derivatives for skin whitening, which inhibit melanin production by suppressing tyrosinase activity. Document CN103957914A describes niacin-peptides with skin whitening activity, achieved through the inhibition of melanogenesis and tyrosinase activity. Additionally, document CN109311938A discusses tranexamic acid-peptide derivatives that also target melanogenesis for skin whitening effects. Overall, these documents focus on inhibiting tyrosinase activity and melanogenesis for achieving skin whitening, but they do not address anti-inflammatory effects or the suppression of inflammatory cytokines such as IL-6 and TNF-α.

Post-inflammatory hyperpigmentation (PIH) is one of the common sequelae of the inflammatory reaction in the skin. At present, the specific pathogenesis of PIH is not completely clear, but as a secondary pigment abnormality, it is closely related to the damage degree to basement membranes, the inflammation level and melanocytes. Bastonini E found that melanocytes and their surrounding keratinocytes and fibroblasts play an important role in the production of PIH. At the same time, the migration, proliferation and differentiation of melanocytes, the synthesis and activation of tyrosinase, and the transfer of melanosomes to keratinocytes can also affect the occurrence of PIH. Some inflammation modulatory factors such as inflammatory mediators, inflammatory cytokines and α-melanocyte-stimulating hormone (α-MSH) also play a role in modulating the above actions to different degrees.

The main method of treating PIH is to control the inflammatory reaction and inhibit melanin synthesis, so as to reduce the persistent pigmentation associated with the micro-inflammatory state. At present, glucocorticoids drug such as desonide and hydrocortisone are mainly used to control the inflammatory reaction. Moreover, other compounds are used to inhibit the synthesis or deposition of melanin. For example, arbutin, kojic acid and ascorbic acid can inhibit melanin synthesis by inhibiting the activity of tyrosinase. Niacin is converted into nicotinamide in vivo and inhibits the transport of melanin granules from melanocytes to keratinocytes. However, these compounds usually have cytotoxicity, skin irritation or chemical instability, and a single compound only has some effect of anti-inflammation or inhibition of melanin generation, and needs to be used in combination. Therefore, there is a need to develop a novel compound, which can not only treat the skin inflammatory reaction but also be used to inhibit melanin hyperpigmentation, thereby overcoming the existing defects in the prior art.

### SUMMARY

The present invention aims to provide a polypeptide derivative with both a whitening effect and anti-inflammatory activity. These peptide derivatives, and cosmetic compositions or pharmaceutical compositions comprising these peptide derivatives can play a whitening role by down-regulating the α-MSH expression and exert anti-inflammatory activity by down-regulating the expressions of IL-6 and TNF-α. They can be used in the fields of cosmetics or medicines for skin anti-inflammation as well as whitening and freckle removing of skin, especially for improving post-inflammation pigmentation.

In this regard, the present invention provides a peptide derivative represented by Formula (I), or a stereoisomer thereof, or a mixture of stereoisomers thereof, or a cosmetically acceptable salt thereof, or a pharmaceutically acceptable salt thereof,

R₁-Pro-Lys-Glu-Lys-R₂ (I)

in Formula (I),
R₁ is selected from substituent (i) or substituent (ii), wherein substituent (i) is 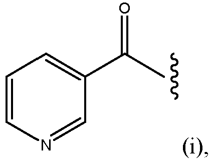
substituent (ii) is 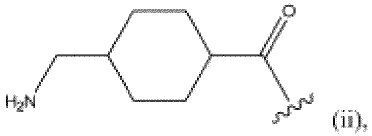
R₂ is selected from -NR₃R₄ or -OR₃, wherein each R₃ and R₄ are independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally is selected from methyl, ethyl, isopropyl, isobutyl, *tert*-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, or optionally has 1, 2, or 3 conjugated or non-conjugated carbon-carbon double bonds; the alkenyl is bonded to the rest portions of a molecule through a single bond, optionally is selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

Optionally, R₃ and R₄ are each independently selected from H, methyl, ethyl, hexyl, dodecyl or hexadecyl;
optionally, R₃ is H and R₄ is selected from H, methyl, ethyl, hexyl, dodecyl or hexadecyl;
optionally, R₂ is -OH or -NH₂.

The peptide derivative represented by Formula (I), or a stereoisomer thereof, or a mixture of stereoisomers thereof, or a cosmetically acceptable salt thereof, or a pharmaceutically acceptable salt thereof, is selected from the following peptide derivatives (1)-(4):

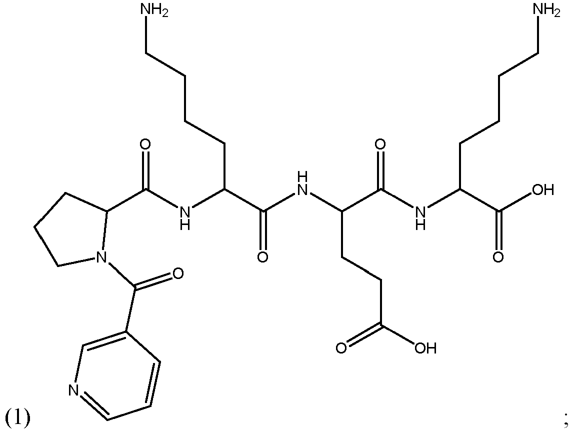

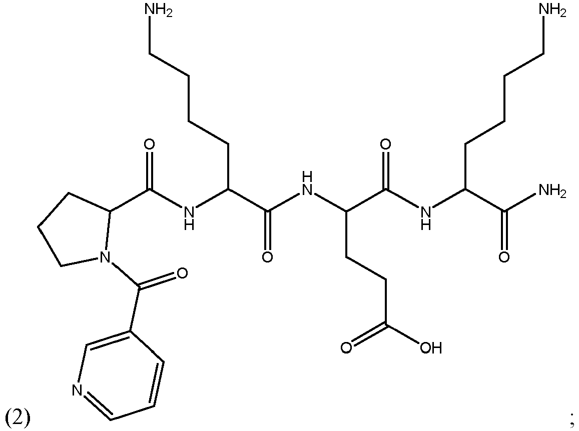

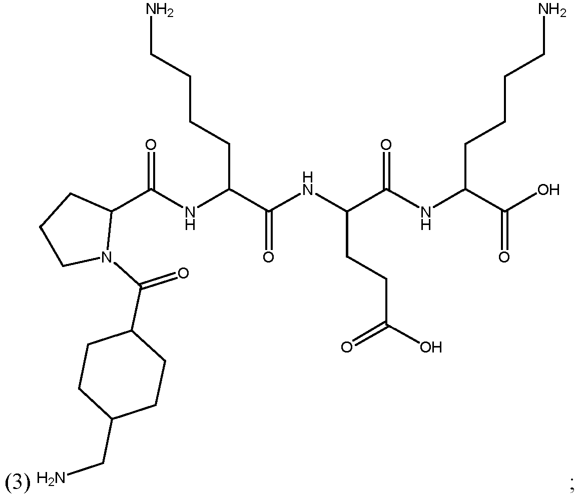

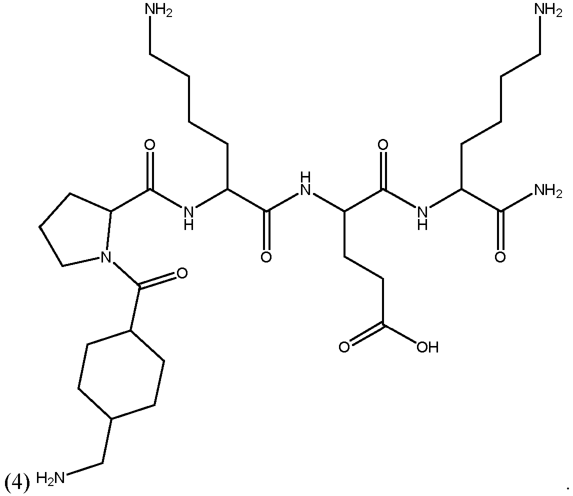

The peptide derivative represented by Formula (I) of the present invention may be present as a stereoisomer or a mixture of stereoisomers thereof; for example, these amino acids comprised therein may have an L- or D-configuration, or be each independently racemic. Thus, it is possible to obtain isomeric mixtures as well as racemic mixtures or diastereomeric mixtures, or pure diastereomers or enantiomers, depending on the number of asymmetric carbons and what isomers or isomeric mixtures are present. Preferred structures of the peptide derivatives represented by Formula (I) of the present invention are pure isomers, i.e., enantiomers or diastereomers.

For example, when -Lys- is mentioned in the present invention, it should be understood that -Lys- is selected from -L-Lys-, -D-Lys-, or a mixture of both, and is racemic or non-racemic. The preparation methods described in this document enable a person skilled in the art to obtain each stereoisomer of the peptide derivatives of the present invention by selecting an amino acid having the correct configuration.

The term "a cosmetically acceptable salt or pharmaceutically acceptable salt" refers to a salt approved for use in animals, and more specifically in humans, including a metal salt of the peptide derivative represented by Formula (I), wherein the metal includes but not limited to lithium, sodium, potassium, calcium, magnesium, manganese, copper, zinc or aluminum, etc.; a salt formed from the peptide derivative represented by Formula (I) and an organic alkali, wherein the organic alkali includes, but not limited to, ethylamine, diethylamine, ethylenediamine, ethanolamine, diethanolamine, arginine, lysine, histidine or piperazine, etc.; a salt formed from the peptide derivative represented by Formula (I) and an inorganic acid or an organic acid, wherein the organic acid includes, but not limited to, acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid, etc.; the inorganic acid includes, but not limited to, hydrochloric acid, sulfuric acid, boric acid or carbonic acid.

Another aspect of the present invention provides a cosmetic or pharmaceutical composition, comprising an effective amount of the peptide derivative represented by Formula (I), or a stereoisomer thereof, or a mixture of stereoisomers thereof, or a cosmetically acceptable salt thereof, or a pharmaceutically acceptable salt thereof, and at least one excipient and optionally a cosmetically or pharmaceutically acceptable adjuvant.

Optionally, the adjuvant is selected from a collagen synthesis stimulator, an agent that modulates the PGC-1α synthesis, an agent that modulates the activity of PPARγ, an agent that increases or decreases a triglyceride content in adipocytes, an agent that stimulates or delays adipocyte differentiation, a lipolytic agent or an agent that simulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, an comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agents derived from a biological fermentation process, an inorganic salt, a cell extract, a sunscreen, and an effective organic or inorganic photoprotectant against A and/or B UV rays, or mixtures thereof.

Optionally, a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or gelatin;
optionally, the capsule includes a soft capsule, a hard capsule, optionally a gelatin capsule;
optionally, the tablet includes a sugar-coated tablet.

The peptide derivatives of the present invention have variable solubilities in water depending on the nature of their sequences or any potential modifications in the N-terminal and/or C-terminal. The peptide derivative of the present invention may therefore be incorporated into a composition via an aqueous solution, and those that are insoluble in water may be soluble in cosmetically or pharmaceutically acceptable conventional solvents, including but not limited to ethanol, propanol, isopropanol, propylene glycol, glycerin, butylene glycol or polyethylene glycol or any combination thereof.

The cosmetically or pharmaceutically effective amounts of the peptide derivatives of the present invention to be administered, as well as doses thereof, will depend on many factors including age, the state of a patient, the severity of a disorder or disease, the route and frequency of administration, and specific properties of the peptide derivatives to be used.

"A cosmetically or pharmaceutically effective amount" refers to an amount of one or more peptide derivatives of the present invention that is nontoxic but sufficient to provide the desired effects. The peptide derivative of the present invention is used in the cosmetic or pharmaceutical composition of the present invention at a cosmetically or pharmaceutically effective concentration to obtain the desired effects. In a preferred form, relative to the total weight of the composition, the concentration is between 0.00000001% (by weight) and 20% (by weight), preferably between 0.000001% (by weight) and 15% (by weight), more preferably between 0.0001% (by weight) and 10 % (by weight), and even more preferably between 0.0001% (by weight) and 5% (by weight).

Another aspect of the present invention provides a cosmetically or pharmaceutically acceptable delivery system or sustained release system, to achieve better penetration of the active ingredient and/or to improve pharmacokinetic and pharmacodynamic properties thereof, which comprises an effective amount of the peptide derivative represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above.

The term "delivery system" refers to a diluent, adjuvant, excipient or carrier administered with the peptide derivative of the present invention, selected from water, oil or surfactants, including those petroleum-derived, animal-derived, plant-derived, or synthesis-derived. Examples include but are not limited to peanut oil, soybean oil, mineral oil, sesame oil, castor oil, polysorbate, sorbitan ester, ether sulfate, sulfate, betaine, glucoside, maltoside, fatty alcohol, nonoxynol, poloxamer, polyoxyethylene, macrogol, dextrose, glycerin, digitonin and the like. Diluents that can be used in the different delivery systems in which the peptide derivative of the present invention can be administered are known to those of ordinary skill in the art.

The term "sustained release" is used in the conventional sense to refer to a delivery system that provides a gradual release of a compound over a period of time, and preferably, but not necessarily, a relatively constant level of compound release over the entire period of time.

Examples of the delivery system or sustained release system include a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle. The preferred delivery system or sustained release system is a liposome and a microemulsion, more preferably a water-in-oil microemulsion having an internal structure of reverse micelles.

The sustained release system can be prepared by methods known in the art and can be administered, for example, by topical or transdermal administration, including adhesive patches, non-adherent patches, sealing patches, and microelectronic patches; or by systemic administration, such as but not limited to, oral or parenteral routes, including nasal, rectal, subcutaneous implantation or injection, or direct implantation or injection to specific body parts. Preferably a relatively constant amount of these peptide derivatives of the present invention should be released. The amount of a peptide derivative comprised in the sustained release system will depend, for example, on the site where the composition is to be administered, the release kinetics and duration of the peptide derivative of the present invention, and the nature of the condition, disorder and/or disease to be treated and/or cared for.

Another aspect of the present invention provides use of the peptide derivative represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above in the preparation of a cosmetic composition or pharmaceutical composition for treatment or care of the skin or mucosa.

Another aspect of the present invention provides use of the peptide derivative represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above in the preparation of a cosmetic composition or pharmaceutical composition for whitening.

Another aspect of the present invention provides use of the peptide derivative represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above in the preparation of a cosmetic composition or pharmaceutical composition for anti-inflammation.

Another aspect of the present invention provides use of the peptide derivative represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above in the preparation of a cosmetic composition or pharmaceutical composition for improving post-inflammation hyperpigmentation.

In order to facilitate understanding of the present invention, the meanings of some terms and expressions used in the present invention are explained as follows.

In the present invention, the term "skin" should be understood to be the layers which comprise it, from the uppermost layer or stratum corneum to the lowermost layer or subcutaneous tissue, both inclusive. These layers are composed of different types of cells such as keratinocytes, fibroblasts, melanocytes and/or adipocytes, among others. In the present invention, the term "skin" includes the scalp.

The term "treatment" means the administration of a peptide derivative of the present invention to alleviate or eliminate a disease or disorder, or reduce or eliminate one or more symptoms associated with the disease or disorder. The term "treatment" also covers the ability to alleviate or eliminate the physiological consequences of the disease or disorder.

The term "care" includes the prevention of a disease and/or disorder.

In the description, the abbreviations used for amino acids follow the rules specified by the IUPAC-IUB Commission of Biochemical Nomenclature in the European Journal of Biochemistry (Eur. J. Biochem. 1984, 138:9-37).

Therefore, for example, Lys represents NH₂-CH(CH₂CH₂CH₂CH₂NH₂)-COOH, Lys-represents NH₂-CH(CH₂CH₂CH₂CH₂NH₂)-CO-, -Lys represents -NH-CH(CH₂CH₂CH₂CH₂NH₂)-COOH, and -Lys- represents -NH-CH(CH₂CH₂CH₂CH₂NH₂)-CO-. Thus, a hyphen representing a peptide bond removes OH in amino acid 1-carboxyl (herein represented in the conventional non-ionized form) when located to the right of the symbol, and removes H in amino acid 2-amino when located to the left of the symbol; both modifications can be applied to the same symbol (see Table 1).

**Table 1 Structures of amino acid residues and one-letter and three-letter abbreviations thereof**

| Name/Abbreviation | Residue | Name/Abbreviation | Residue |
|---|---|---|---|
| Prolyl | 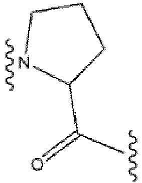 | Lysyl | 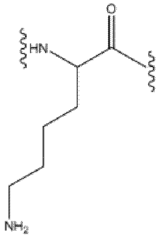 |
| -Pro- | | -Lys- | |
| P | | K | |
| Glutamyl | 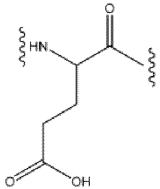 | | |
| -Glu- | | | |
| E | | | |

The beneficial effects that the present invention achieves over the prior art include:
1. The peptide derivative of the present invention is obtained through artificial design and is easy to synthesize.
2. The N-terminal of H-Pro-Lys-Glu-Lys-OH is covalently linked to niacin or tranexamic acid to form a newly structured compound that can down-regulate the α-MSH expression, inhibit the melanin generation, exerts a whitening effect, and has higher whitening activity than a single niacin, tranexamic acid or kojic acid, and a single polypeptide H-Pro-Lys-Glu-Lys-OH.
3. The N-terminal of H-Pro-Lys-Glu-Lys-OH is covalently linked to niacin or tranexamic acid to form a newly structured compound that can down-regulate the expressions of IL-6 and TNF-α and exhibits unexpected anti-inflammatory activity based on the whitening effect.
4. The peptide derivative of the present invention has both a whitening effect and anti-inflammatory activity and can be used in the fields of cosmetics or medicines for anti-inflammation as well as whitening and freckle removing of skin, especially for improving post-inflammation pigmentation.

### BRIEF DESCRIPTION OF THE DRAWINGS

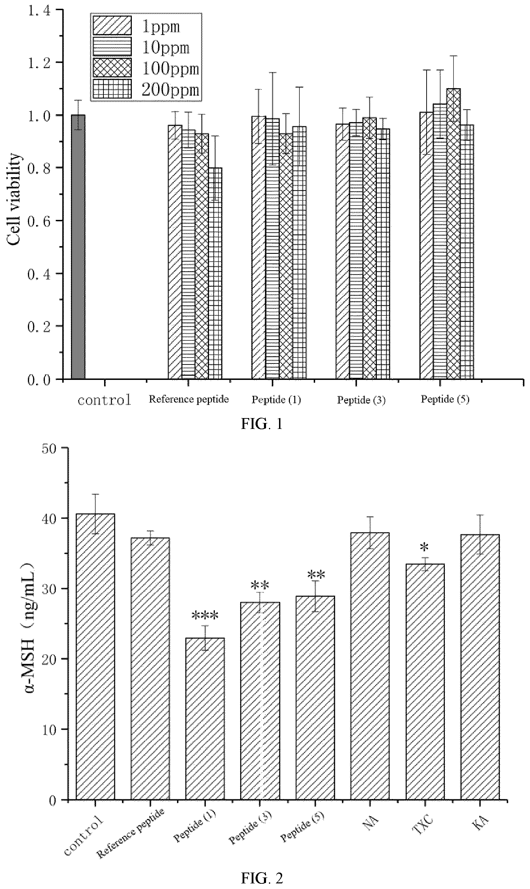

FIG. 1 is a graph showing the effect of the peptide derivatives of the present invention on the HaCaT cell viability (n=4).
FIG. 2 is a graph showing the effect results of 100 ppm of the peptide derivatives of the present invention on α-MSH expression. * indicates a statistical difference between the drug group and the control group, p<0.05 (n=4). ** indicates a significant difference between the drug group and the control group, p<0.01 (n=4). *** indicates an extremely significant difference between the drug group and the control group, p<0.001 (n=4).

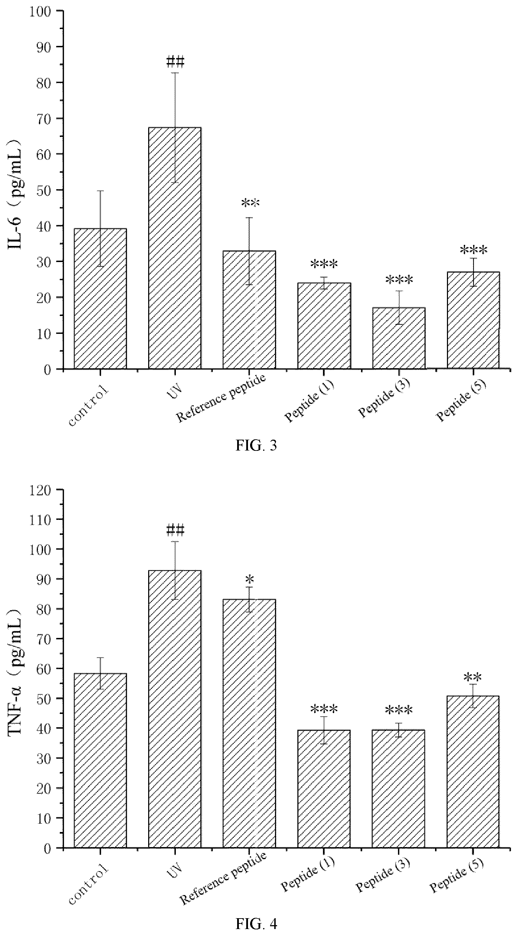

FIG. 3 is a graph showing the effect results of 50 ppm of the peptide derivatives of the present invention on IL-6 expression. ## indicates a significant difference between the UV group and the control group, p<0.01 (n=4). ** indicates a significant difference between the drug group and the UV group, p<0.01 (n=4). *** indicates an extremely significant difference between the drug group and the UV group, p<0.001 (n=4).
FIG. 4 is a graph showing the effect results of 50 ppm of the peptide derivatives of the present invention on TNF-α expression. ## indicates a significant difference between the UV group and the control group, p<0.01 (n=4). * indicates a statistical difference between the drug group and the UV group, p<0.05 (n=4). ** indicates a significant difference between the drug group and the UV group, p<0.01 (n=4). *** indicates an extremely significant difference between the drug group and the UV group, p<0.001 (n=4).

### DETAILED DESCRIPTION OF THE EMBODIMENTS

In order to better understand the present invention, the invention will be described in detail below in conjunction with examples and drawings. However, it should be understood that these examples and drawings are only used for illustration purposes, and are not intended to limit the scope of the present invention.

### Abbreviations

The abbreviations used for amino acids follow the rules specified by the Biochemical Nomenclature Commission of IUPAC-IUB in Eur J. Biochem. (1984) 138:9-37 and J. Chem (1989) 264:633-673.

2-Cl-Trt resin: 2-chlorotrityl resin, a starting resin for polypeptide synthesis (crosslinking degree 1%, substitution degree 1.24 mmol/g); Trt: trityl; DMF: N,N-dimethylformamide; DCM: dichloromethane; DIPEA: diisopropylethylamine; DIC: diisopropylcarbodiimide; piperidine: piperidine; HOBt: 1-hydroxybenzotriazole; TFA: trifluoroacetic acid; TIS: triisopropylsilane; Pro: proline; Lys: lysine; Glu: glutamate; Fmoc: 9-fluorenylmethoxycarbonyl; Boc: *tert*-butoxycarbonyl; OtBu: *tert*-butoxyl; CDI: N,N'-carbonyldiimidazole; Py: pyridine;

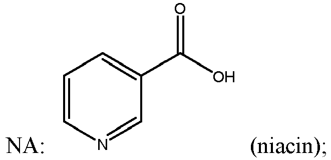

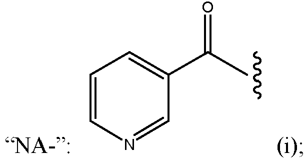

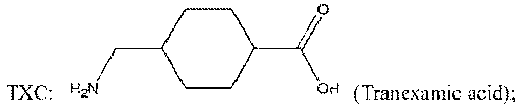

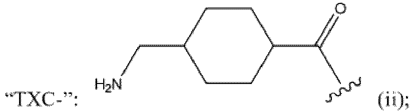

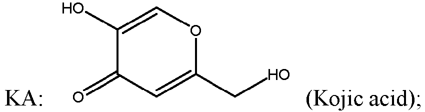

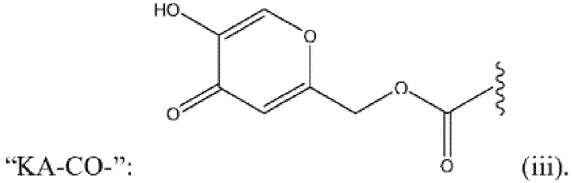

According to the amino acid structure of the carboxyl-terminal of a polypeptide sequence, the corresponding resin is chosen as a carrier for solid-phase synthesis.

The resin is added to swell and washed with a solvent. Activated amino acids are added. Under the action of the coupling system, the resin is linked to the amino acids in order from the carboxyl-terminal to the amino-terminal in a coupling solvent according to the peptide sequence, and the resin is washed to obtain a peptidyl resin.

Specifically, Fmoc-Lys(Boc)-OH, Fmoc-Glu(OtBu)-OH, Fmoc-Lys(Boc)-OH, and Fmoc-Pro-OH are added in order to react until all amino acids are linked. The reaction product is washed with dichloromethane followed by drying, to afford a Fmoc-Pro-Lys(Boc)-Glu(OtBu)-Lys(Boc)-resin, which is divided into three batches.

One batch of the above-mentioned peptidyl resin is taken and coupled with NA to afford a NA-Pro-Lys(Boc)-Glu(OtBu)-Lys(Boc)-resin, which is subjected to cleavage, concentration, sedimentation, centrifugation, and vacuum drying to afford a crude peptide; the crude peptide is purified by reversed-phase high-performance liquid chromatography and freeze-dried to afford NA-Pro-Lys-Glu-Lys-OH or NA-Pro-Lys-Glu-Lys-NH₂.

One batch of the above-mentioned peptidyl resin is taken and coupled with Fmoc-TXC-OH to afford a Fmoc-TXC-Pro-Lys(Boc)-Glu(OtBu)-Lys(Boc)-resin, which is subjected to cleavage, concentration, sedimentation, centrifugation, and vacuum drying to afford a crude peptide; the crude peptide is purified by reversed-phase high-performance liquid chromatography and freeze-dried to afford TXC-Pro-Lys-Glu-Lys-OH or TXC-Pro-Lys-Glu-Lys-NH₂.

KA cannot directly form an amide bond with the N-terminal of the polypeptide, so in the presence of Py and DMF, KA reacts with CDI to form a KA-CO-imidazole intermediate, and then covalently binds to the N-terminal of the polypeptide through the intermediate.

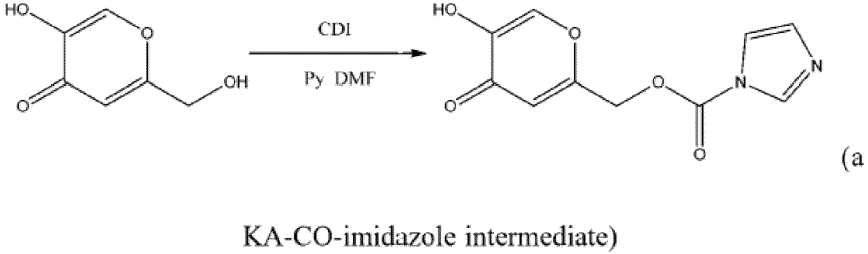

One batch of the above-mentioned peptidyl resin is taken and coupled with the KA-CO-imidazole intermediate to afford a KA-CO-Pro-Lys(Boc)-Glu(OtBu)-Lys(Boc)-resin, which is subjected to cleavage, concentration, sedimentation, centrifugation, and vacuum drying to afford a crude peptide; the crude peptide is purified by reversed-phase high-performance liquid chromatography and freeze-dried to afford KA-CO-Pro-Lys-Glu-Lys-OH or KA-CO-Pro-Lys-Glu-Lys-NH₂.

### EXAMPLE 1

### Preparation of Fmoc-Pro-Lys(Boc)-Glu(OtBu)-Lys(Boc)-2-Cl-Trt resin

### 1.1 Swelling of resin

200 ml DMF was used to spray column wall and drained for 3 min. 200 ml DMF was used to spray the column wall again and drained for 5 min. 20 g a 2-Cl-Trt resin having a substitution degree of 1.42 mmol/g was added into a solid phase column, 200 ml DMF was added to completely immerse the resin followed by nitrogen purge and stirring at 100 r/min for 5 min, and then the solvent was drained for 5 min. 200 ml DMF was added again to completely immerse the resin followed by nitrogen purge and stirring at 100 r/min for 30 min, and then the solvent was drained. 200 ml DMF was further added to completely immerse the resin followed by nitrogen purge and stirring at 100r/min, and then the solvent was drained for 5 min to complete swelling.

### 1.2 Fmoc deprotection

200 ml 20% piperidine/DMF was added with nitrogen purge, stirred and reacted for 5 min, and drained for 3 min. 200 ml 20% piperidine/DMF was added again with nitrogen purge, stirred for 8 min, and drained for 3 min. Then 200 ml DMF was added with a nitrogen purge, stirred for 1-2 min, and drained for 3 min. The resin was washed repeatedly 7-8 times.

### 1.3 Feeding reaction

17.4 g Fmoc-Lys(Boc)-OH was dissolved in 100 ml DMF comprising 4.3 ml DIPEA (1.5 eq) and added to a solid phase reactor to couple with the 2-Cl-Trt resin in a reaction for 14 h. After being filtered and washed, the reaction product was treated with methanol for 1 h to block the remaining chloride groups.

The substitution degree was detected to be 0.55 mmol/g, and the synthesis scale was determined to be 16.7 mmol. On this basis, the amounts of the remaining amino acid raw materials and agents are calculated as shown in Table 2 below.

**Table 2 Amount of each amino acid raw material and agent**

| No. | Name of amino acid raw material | Amount (g) | Multiple of synthesis scale | HOBt (g) | DIC (g) |
|---|---|---|---|---|---|
| 1 | Fmoc-Glu(OtBu)-OH | 17.8 | 2.5 | 6.8 | 7 |
| 2 | Fmoc-Lys(Boc)-OH | 19.5 | 2.5 | 6.8 | 7 |
| 3 | Fmoc-Pro-OH | 14.1 | 2.5 | 6.8 | 7 |

The N-terminal Fmoc group was deprotected, and in the presence of 6.8 g HOBt and 7 g DIC, 17.8 g of the activated Fmoc-Glu(OtBu)-OH were coupled to peptidyl resins using DMF as solvent. The reaction lasted 2.5 h. The resins were then washed and repeatedly subjected to the deprotection treatment of the Fmoc group so as to couple to the next amino acid.

In each coupling, in the presence of 6.8 g HOBt and 7 g DIC, and DMF as a solvent, the resin was sequentially coupled to 19.5 g Fmoc-Lys(Boc)-OH, and 14.1 g Fmoc-Pro-OH.

### 1.4 Washing the resin after the completion of the reaction

After the synthesis, the resin was washed with DMF, each time 100 ml. The resin and the solvent were evenly mixed and then stirred for 2 min. The solvent was drained. The resin was washed with DMF many times until the filtered solvent was clear and transparent. After shrinkage and drying, a Fmoc-Pro-Lys(Boc)-Glu(OtBu)-Lys(Boc)-2-Cl-Trt resin was obtained.

### EXAMPLE 2

### Preparation of NA-Pro-Lys-Glu-Lys-OH

### 2.1 Preparation of NA-Pro-Lys(Boc)-Glu(OtBu)-Lys(Boc)-2-Cl-Trt resin

10 g Fmoc-Pro-Lys(Boc)-Glu(OtBu)-Lys(Boc)-2-Cl-Trt resin was weighed and added to a 100 ml solid phase column, and washed with DMF three times after swelling with DMF. Fmoc was deprotected using a 20% piperidine/DMF solution, followed by washing and a K-test (a 5% ethanol solution of ninhydrin) showing a color of dark blue.

In the presence of 2.65 g DIC and 2.27 g HOBt, 1.71 g NA reacted with the above-mentioned Fmoc-deprotected peptidyl resin for 2 h, and the K test showed that the resin was colorless and transparent. The resin was washed three times with DMF, 30 ml each time, and washed twice with DCM, 30 ml each time. The resin was shrunk and dried with 30 ml of methanol to afford a NA-Pro-Lys(Boc)-Glu(OtBu)-Lys(Boc)-2-Cl-Trt resin.

### 2.2 Preparation of NA-Pro-Lys-Glu-Lys-OH

### 2.2.1 Preparation of a lysis buffer

24.75 ml TFA, 1.5 ml TIS, and 1.5 ml water were measured, mixed and stirred, followed by placing in a -18°C refrigerator for subsequent use.

### 2.2.2 Cleavage

10 g of the NA-Pro-Lys(Boc)-Glu(OtBu)-Lys(Boc)-2-Cl-Trt resin was weighed and added to a 100 ml round-bottom flask, and 30 ml of the frozen lysis buffer were added to react with stirring for 2 h. After the reaction product was filtered and concentrated to 10 ml, isopropyl ether was added to precipitate a white solid, which was washed with isopropyl ether six times until the pH value was 3-4, and vacuum dried. 6 g of a crude peptide (purity 85%) was obtained.

### 2.2.3 Purification

6 g of the crude peptide were weighed and dissolved in 85 ml pure water to afford a light yellow liquid, which was filtered with a 0.22 µm microporous filter membrane to afford a light yellow clear and transparent solution. The solution was subjected to reversed-phase HPLC purification. The purification gradient is as follows:

| Time (min) | Flow rate (ml/min) | A% (0.1% acetic acid + acetonitrile) | B% (0.1% acetic acid + pure water) |
|---|---|---|---|
| 0 | 15 | 2 | 98 |
| 20 | 15 | 10 | 90 |
| 30 | 15 | 20 | 80 |
| 35 | 15 | 50 | 50 |
| 40 | 15 | 50 | 50 |

The filtered sample was injected and purified. Fractions were collected, concentrated and freeze-dried to afford the peptide (1) NA-Pro-Lys-Glu-Lys-OH having a purity of 97.97%.

### EXAMPLE 3

### Preparation of TXC-Pro-Lys-Glu-Lys-OH

### 3.1 Preparation of TXC-Pro-Lys(Boc)-Glu(OtBu)-Lys(Boc)-2-Cl-Trt resin

10 g of the Fmoc-Pro-Lys(Boc)-Glu(OtBu)-Lys(Boc)-2-Cl-Trt resin was weighed and added to a 100 ml solid phase column, and washed with DMF three times after swelling with DMF. Fmoc was deprotected using a 20% piperidine/DMF solution, followed by washing and a K-test (a 5% ethanol solution of ninhydrin) showing a color of dark blue.

In the presence of 2.65 g DIC and 2.27 g HOBt, 5.31 g Fmoc-TXC-OH reacted with the above-mentioned Fmoc-deprotected peptidyl resin for 2 h, and the K test showed that the resin was colorless and transparent. Fmoc was deprotected using a 20% piperidine/DMF solution. The resin was washed three times with DMF, 30 ml each time, and washed twice with DCM, 30 ml each time. The resin was shrunk and dried with 30 ml methanol to afford a TXC-Pro-Lys(Boc)-Glu(OtBu)-Lys(Boc)-2-Cl-Trt resin.

### 3.2 Preparation of TXC-Pro-Lys-Glu-Lys-OH

### 3.2.1 Preparation of a lysis buffer

24.75 ml TFA, 1.5 ml TIS, and 1.5 ml water were measured, mixed and stirred, followed by placing in a -18°C refrigerator for subsequent use.

### 3.2.2 Cleavage

10 g of the TXC-Pro-Lys(Boc)-Glu(OtBu)-Lys(Boc)-2-Cl-Trt resin was weighed and added to a 100 ml round-bottom flask, and 30 ml of the frozen lysis buffer was added to react with stirring for 2 h. After the reaction product was filtered and concentrated to 10 ml, isopropyl ether was added to precipitate a white solid, which was washed with isopropyl ether six times until the pH value was 3-4, and vacuum dried. 5.7 g a crude peptide (purity 87%) was obtained.

### 3.2.3 Purification

5.7 g of the crude peptide was weighed and dissolved in 85 ml pure water to afford a light yellow liquid, which was filtered with a 0.22 µm microporous filter membrane to afford a light yellow clear and transparent solution. The solution was subjected to reversed-phase HPLC purification. The purification gradient is as follows:

| Time (min) | Flow rate (ml/min) | A% (0.1% acetic acid + acetonitrile) | B% (0.1% acetic acid + pure water) |
|---|---|---|---|
| 0 | 15 | 15 | 85 |
| 15 | 15 | 25 | 75 |
| 16 | 15 | 60 | 40 |
| 21 | 15 | 60 | 40 |
| 22 | 15 | 15 | 85 |

The filtered sample was injected and purified. Fractions were collected, concentrated and freeze-dried to afford the peptide (3) TXC-Pro-Lys-Glu-Lys-OH having a purity of 96.50%.

### EXAMPLE 4 (not part of the present invention)

### Preparation of KA-CO-Pro-Lys-Glu-Lys-OH

### 4.1 Synthesis of a KA-CO-imidazole intermediate

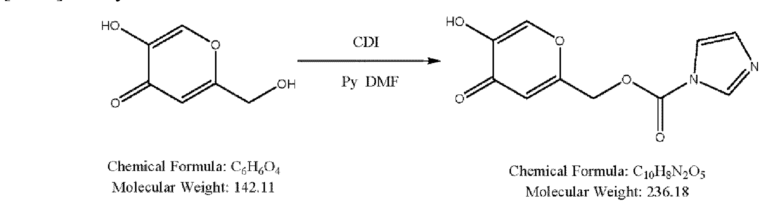

In a 250ml three-necked flask, KA (50 g, 35.2 mmol) was added and dissolved in 150 ml DMF; Py (8 g, 100 mmol) was added; the temperature was lowered to 0°C; CDI (6.6 g, 40 mmol) was added to and reacted for 4 h. By TLC analysis the reaction was complete (developing agent: MeOH:DCM=1:20, Rf value 0.67).

The reaction solution was poured into ice water, and a large amount of a white solid precipitated. After filtration, the solid was washed with ice water until there was no odor, and vacuum dried to afford 74 g of a product with a yield of 89.05%.

### 4.2 Preparation of KA-CO-Pro-Lys(Boc)-Glu(OtBu)-Lys(Boc)-2-Cl-Trt resin

10 g of the Fmoc-Pro-Lys(Boc)-Glu(OtBu)-Lys(Boc)-2-Cl-Trt resin was weighed and added to a 100 ml solid phase column, and washed with DMF three times after swelling with DMF. Fmoc was deprotected using a 20% piperidine/DMF solution, followed by washing and a K-test (a 5% ethanol solution of ninhydrin) showing a color of dark blue.

In the presence of 3.62 g DIPEA and, 3.31 g KA-CO-imidazole intermediate reacted with the above-mentioned Fmoc-deprotected peptidyl resin for 2 h, and the K test showed that the resin was colorless and transparent. The resin was washed three times with DMF, 30 ml each time, and washed twice with DCM, 30 ml each time. The resin was shrunk and dried with 30 ml methanol to afford a KA-CO-Pro-Lys(Boc)-Glu(OtBu)-Lys(Boc)-2-Cl-Trt resin.

### 4.3 Preparation of KA-CO-Pro-Lys-Glu-Lys-OH

### 4.3.1 Preparation of a lysis buffer

24.75 ml TFA, 1.5 ml thioanisole, 1.5 ml phenol, 1.5 ml water, and 0.75 ml EDT were measured, mixed and stirred, followed by placing in a -18°C refrigerator for subsequent use.

### 4.3.2 Cleavage

10 g of the KA-CO-Pro-Lys(Boc)-Glu(OtBu)-Lys(Boc)-2-CI-Trt resin was weighed and added to a 100 ml round-bottom flask, and 30 ml of the frozen lysis buffer was added to react with stirring for 2 h. After the reaction product was filtered and concentrated to 10 ml, isopropyl ether was added to precipitate a white solid, which was washed with isopropyl ether six times until the pH value was 3-4, and vacuum dried. 5.7 g of a crude peptide (purity 83.5%) was obtained.

### 4.3.3 Purification

5.7 g of the crude peptide was weighed and dissolved in 65 ml pure water to afford a light yellow liquid, which was filtered with a 0.22 µm microporous filter membrane to afford a light yellow clear and transparent solution. The solution was subjected to reversed-phase HPLC purification. The purification gradient is as follows:

| Time (min) | Flow rate (ml/min) | A% (0.1% acetic acid + acetonitrile) | B% (0.1% acetic acid + pure water) |
|---|---|---|---|
| 0 | 15 | 0 | 100 |
| 30 | 15 | 27 | 75 |
| 31 | 15 | 50 | 50 |
| 35 | 15 | 50 | 50 |

The filtered sample was injected and purified. Fractions were collected, concentrated and freeze-dried to afford the peptide (5) KA-CO-Pro-Lys-Glu-Lys-OH having a purity of 97.34%.

### EXAMPLE 5

Other compounds of Formula (I) of the present invention can be prepared in similar processes.

The molecular weight of the obtained peptide derivatives was determined by ESI-MS. The test results of some compounds are shown in Table 3 below.

**Table 3 Determination of molecular weight by mass spectrometry**

| No. | Sequence | Theoretical molecular weight | Molecular weight mass spectrometry analysis results |
|---|---|---|---|
| (1) | NA-Pro-Lys-Glu-Lys-OH | 605.69 | 605.45 |
| (3) | TXC-Pro-Lys-Glu-Lys-OH | 639.80 | 639.41 |
| (5) | KA-CO-Pro-Lys-Glu-Lys-OH* | 668.70 | 668.30 |

| | | | |
|---|---|---|---|
| *not according to the present invention | | | |

### EXAMPLE 6

Cell viability assay

### 6.1 Reagents and materials

Phosphate-buffered saline (PBS) (Gibco), thiazolyl blue (MTT) (Sigma), dimethyl sulfoxide (DMSO) (Sigma), a high glucose culture medium (DMEM) (Gibco), and fetal bovine serum (Gibco).

### 6.2 Instruments

A microplate reader (MD, USA), a CO₂ incubator (Shanghai Yiheng), and an ultra-clean workbench (Suzhou Purification).

### 6.3 Cell lines

Human keratinocytes (HaCaT) were purchased from the Kunming Cell Bank of China Center for Type Culture Collection under the Chinese Academy of Sciences.

### 6.4 Samples to be tested

### Drug groups:

the peptide H-Pro-Lys-Glu-Lys-OH (hereinafter referred to as the reference peptide), at a test concentration of 1 ppm, 10 ppm, 100 ppm, and 200 ppm, respectively;
the peptide (1), at a test concentration of 1 ppm, 10 ppm, 100 ppm, and 200 ppm, respectively;
the peptide (3), at a test concentration of 1 ppm, 10 ppm, 100 ppm, and 200 ppm, respectively;
the peptide (5), at a test concentration of 1 ppm, 10 ppm, 100 ppm, and 200 ppm, respectively.

### Control group:

PBS blank control.

### 6.5 Experiment purpose

The purpose of this experiment is to evaluate cell viability 72 h after administration and determine the effect of the peptide derivatives of the present invention on cell viability by using HaCaT cells as the experiment subject.

### 6.6 Experimental protocol

The frozen HaCaT cells were cultured and passaged at a ratio of 1:2 to about the 5th generation, and the cells in better growth were selected as experiment subjects.

The cells were inoculated in a 96-well plate at 2000 cells/well. After the cells adhered to the wall, samples of the drug groups and the control group were added according to the multiple dilution method, supplemented with a culture medium to 200 µL, and cultured in a 5% CO₂ incubator at 37 °C for 72 h.

Then 20 µL 5 mg/ml MTT was added to each well and the incubation was continued in the 5% CO₂ incubator at 37°C for 4 h. The original solution was removed and DMSO was added at 150 µL/well. After 5 min, the microplate reader was used to read the reference OD values at 490nm and 630nm wavelengths.

### 6.7 Results

The MTT assay is a method of detecting cell survival and growth. The measured OD values are directly proportional to cell viability.

FIG. 1 showed the test results of HaCaT cell viability. The results showed that compared with the control group, the peptide (1), the peptide (3) and the peptide (5) had no significant effect on HaCaT cell viability, indicating that the peptide derivatives of the present invention had no toxicity to HaCaT cells at an amount in a range of 0-200 ppm.

### Example 7

Cell assay for inhibition of α-MSH generation

### 7.1 Reagents and materials

Fetal bovine serum, DMEM medium, penicillin, streptomycin, and an α-MSH ELISA kit.

### 7.2 Instruments

A microplate reader, a CO₂ incubator, and a constant temperature shaker.

### 7.3 Cell lines

Human keratinocytes (HaCaT) were purchased from the Kunming Cell Bank of China Center for Type Culture Collection under the Chinese Academy of Sciences.

### 7.4 Samples to be tested

### Drug groups:

100 ppm of the reference peptide, 100 ppm of the peptide (1), 100 ppm of the peptide (3), 100 ppm of the peptide (5), 100 ppm of NA, 100 ppm of TXC, and 100 ppm of KA.

### Control group:

PBS blank control.

### 7.5 Experimental protocol

The frozen HaCaT cells were cultured and passaged at a ratio of 1:2 to about the 5th generation, and the cells in better growth were selected as experiment subjects.

When the cells were about 80% confluent, the samples were added to each group. In the control group, a PBS solution was added and supplemented with a culture medium to 1000 µL. In the drug groups, the diluted active substance and the culture medium were added to 1000 µL and the groups were further incubated in a 5% CO₂ incubator at 37°C for 48 h. After the completion of the culture, the solutions were centrifuged at 3000 rpm for 10 min, in each of which a cell supernatant was collected to obtain a sample liquid. Operations were performed according to the instructions of the α-MSH kit.

### 7.6 Results

α-MSH can promote the formation of melanin by activating tyrosinase. Therefore, down-regulation of the α-MSH expression can reduce melanin generation and exert a whitening effect.

The effect results of 100 ppm of the peptide derivatives of the present invention on α-MSH expression were shown in FIG. 2. The results showed that compared with the control group, the reference peptide, NA, TXC, and KA all inhibited the α-MSH expression to a certain extent. However, except TXC, the reference peptide, NA, and KA at 100 ppm had no statistical difference in the α-MSH inhibition compared with the control group.

In contrast, the newly structured compounds peptide (1), peptide (3), and peptide (5) obtained by covalently linking the N-terminal of the reference peptide to NA, TXC, and KA respectively significantly down-regulated the α-MSH expression and had higher activity than NA, TXC or KA alone, or the reference peptide alone, producing unexpected technical effects, wherein the peptide (1) had the strongest inhibitory effect on α-MSH. It was hence indicated that the peptide derivatives of the present invention significantly down-regulated the α-MSH expression, thereby inhibiting melanin production, and had a better whitening effect than NA, TXC or KA alone, or the reference peptide alone.

### EXAMPLE 8

Anti-inflammatory cell assay

### 8.1 Reagents and materials

Fetal bovine serum, DMEM medium, penicillin, streptomycin, an IL-6 ELISA kit and a TNF-α ELISA kit.

### 8.2 Instruments

A microplate reader, a constant temperature CO₂ incubator, and a constant temperature shaker.

### 8.3 Cell lines

Human keratinocytes (HaCaT) were purchased from the Kunming Cell Bank of China Center for Type Culture Collection under the Chinese Academy of Sciences.

### 8.4 Samples to be tested

### Drug groups:

50 ppm of the reference peptide, 50 ppm of the peptide (1), 50 ppm of the peptide (3), and 50 ppm of the peptide (5).

### Control group:

PBS blank control.

### 8.5 Experimental protocol

The frozen HaCaT cells were cultured and passaged at a ratio of 1:2 to about the 5th generation, and the cells in better growth were selected as experiment subjects.

When the cells were about 80% confluent, a model of inflammation stimulated by UV radiation was established. In the control group, a PBS solution was added and supplemented with the culture medium to 1000 µL, while no UV irradiation was performed; The UV group and the drug groups were exposed to an 80 J/cm³ UV light. After irradiation, in the UV group the PBS solution and a culture medium were added to 1000 µL, and in the drug groups the diluted active substance and the culture medium were added to 1000 µL. The control group, the UV group, and the drug groups were further incubated in a 5% CO₂ incubator at 37°C for 48 h. After the completion of the culture, the solutions were centrifuged at 3000 rpm for 10 min, in each of which a cell supernatant was collected to obtain a sample liquid. Operations were performed according to the instructions of the IL-6 and TNF-α kits.

### 8.6 Results

Ultraviolet radiation can stimulate the inflammatory reaction of the skin, leading to increased secretion of TNF-α and IL-6. Therefore, inhibiting the secretion of inflammatory factors such as IL-6 and TNF-α can exert an anti-inflammatory effect.

The effect results of 50 ppm of the peptide derivatives of the present invention on the expression of inflammatory factors such as IL-6 and TNF-α were shown in FIGS. 3 and 4 respectively. The results in FIG. 3 showed that compared with the control group, IL-6 increased significantly in the UV group after ultraviolet irradiation. After administration, the reference peptide, the peptide (1), the peptide (3), and the peptide (5) all significantly down-regulated the IL-6 expression, and the drug groups of the peptide (1), the peptide (3), and the peptide (5) were greatly significantly different from the UV group and had higher anti-inflammatory activity than the reference peptide.

The results in FIG. 4 showed that compared with the control group, TNF-α increased significantly in the UV group after ultraviolet irradiation. After administration, the reference peptide inhibited the TNF-α expression to a certain extent, but the inhibitory effect was not obvious; the peptide (1), the peptide (3), and the peptide (5) all significantly down-regulated the TNF-α expression, and had higher anti-inflammatory activity than the reference peptide.

In summary, the newly structured compounds peptide (1) and peptide (3) obtained by covalently linking the N-terminal of the reference peptide to NA and TXC respectively significantly down-regulated the expressions of IL-6 and TNF-α, and had better effects than the reference peptide alone, showing unexpected anti-inflammatory activity on the basis of a whitening effect. It was hence indicated that the peptide derivatives of the present invention had both a whitening effect and anti-inflammatory activity, and could be used in the fields of cosmetics or medicines for anti-inflammation as well as whitening and freckle removing of skin, especially for improving post-inflammation hyperpigmentation.

### EXAMPLE 9

### Preparation of a skin toner comprising the peptide (1)

| | |
|---|---|
| Ingredients | by weight % |
| Propylene glycol | 1.0 |
| Allantoin | 0.3 |
| Glycerin | 1.0 |
| PEG-7 glyceryl cocoate | 1.0 |
| Peptide (1) | 0.005 |
| Preservative | 0.5 |
| Perfume | 0.5 |
| Water | Balance to 100 |

Allantoin and glycerin were dissolved in water and heated to 85°C at which the temperature was kept for 30 min; PEG-7 glyceryl cocoate and the peptide (1) were dissolved in water; the above solutions were mixed after cooling, and stirred evenly to give a mixed solution; propylene glycol, a preservative, and perfume were added to the above mixed solution in turn, and water was added with stirring evenly to afford a skin toner.

### EXAMPLE 10

### Preparation of a cleansing gel comprising peptide (3)

| Ingredients | by weight % |
|---|---|
| Carbomer (2% emulsion) | 25.0 |
| Triethanolamine | 0.5 |
| Peptide (3) | 0.005 |
| Preservative | 0.2 |
| EDTA | 0.1 |
| Perfume | 0.5 |
| Water | Balance to 100 |

A peptide (3) was dissolved in water and stirred evenly to give a polypeptide solution; carbomer and EDTA were dissolved in water and heated to 85°C; the temperature was kept for 30 min; after cooling triethanolamine was added and stirred evenly to give a mixed solution; the above mixed solution, the polypeptide solution, a preservative, and perfume were added to water in turn and stirred until completely swollen to afford a cleansing gel.

The above contents are further detailed descriptions of the present invention in conjunction with specific preferred embodiments, but it is not indicated that the specific embodiments of the present invention are limited to these descriptions. For a person of ordinary skill in the art of the present invention, without departing from the concept of the present invention, some simple deductions or substitutions to be made should be regarded as belonging to the protection scope of the present invention.

## Claims

1. A peptide derivative represented by Formula (I), or a stereoisomer thereof, or a mixture of stereoisomers thereof, or a cosmetically acceptable salt thereof, or a pharmaceutically acceptable salt thereof,
R₁-Pro-Lys-Glu-Lys-R₂ (I)
in Formula (I),
R₁ is selected from substituent (i) or substituent (ii), wherein substituent (i) is 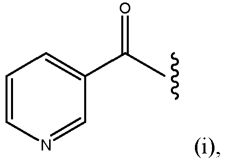
substituent (ii) is 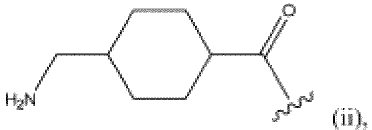
R₂ is selected from -NR₃R₄ or -OR₃, wherein each R₃ and R₄ are independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or having optionally 1, 2, 3, 4, 5, or 6 carbon atoms); optionally is selected from methyl, ethyl, isopropyl, isobutyl, *tert*-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, or optionally has 1, 2, or 3 conjugated or non-conjugated carbon-carbon double bonds; the alkenyl is bonded to the rest portions of a molecule through a single bond, optionally is selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

2. The peptide derivative represented by Formula (I) according to claim 1, or a stereoisomer thereof, or a mixture of stereoisomers thereof, or a cosmetically acceptable salt thereof, or a pharmaceutically acceptable salt thereof, **characterized in that** R₃ and R₄ are independently selected from H, methyl, ethyl, hexyl, dodecyl or hexadecyl;
optionally, R₃ is H and R₄ is selected from H, methyl, ethyl, hexyl, dodecyl or hexadecyl;
optionally, R₂ is -OH or -NH₂.

3. The peptide derivative represented by Formula (I) according to any of claims 1-2, or a stereoisomer thereof, or a mixture of stereoisomers thereof, or a cosmetically acceptable salt thereof, or a pharmaceutically acceptable salt thereof, **characterized in that** it is selected from the following peptide derivatives (1)-(4):

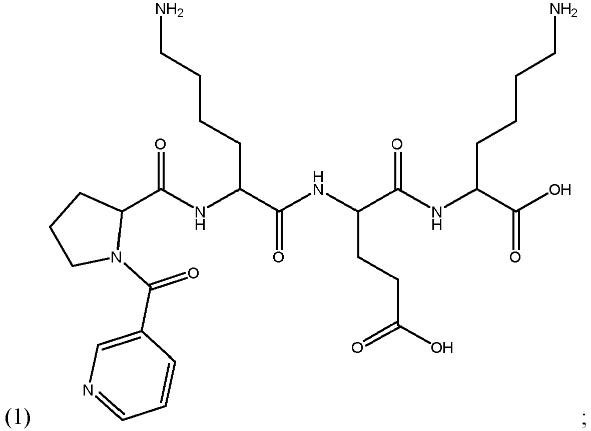

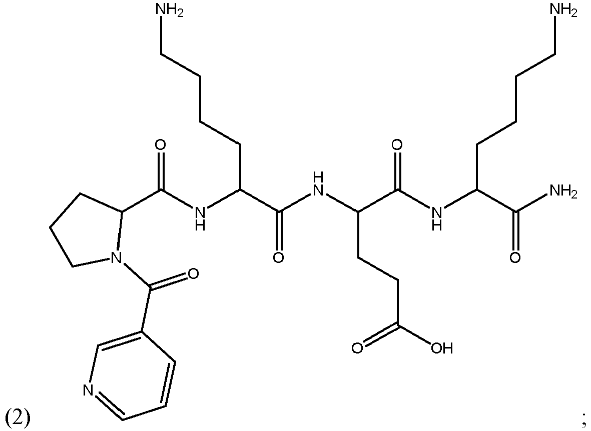

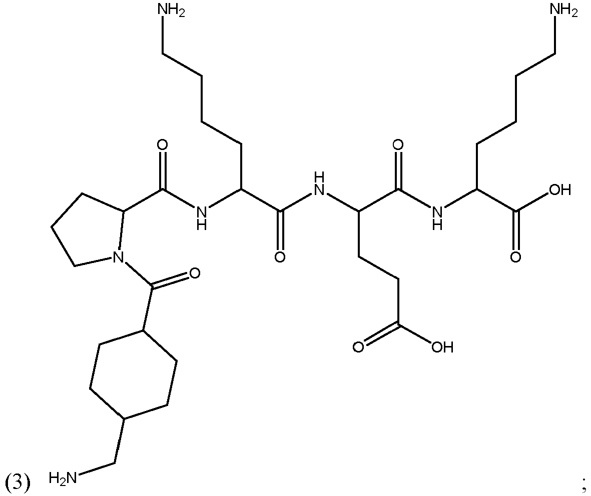

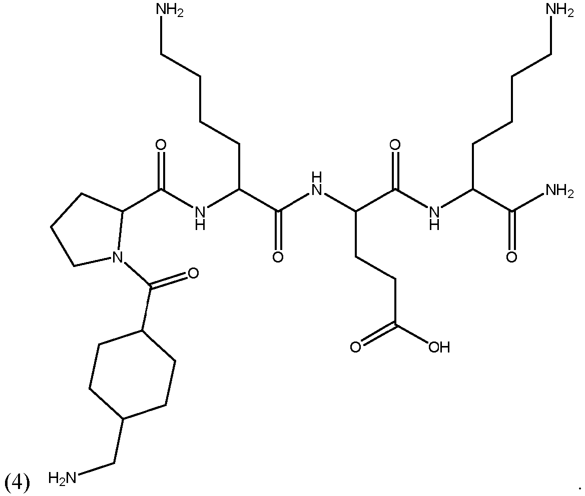

4. The peptide derivative represented by Formula (I) according to claims 1, or a stereoisomer thereof, or a mixture of stereoisomers thereof, or a cosmetically acceptable salt thereof, or a pharmaceutically acceptable salt thereof, **characterized in that**
the cosmetically acceptable salt or pharmaceutically acceptable salt includes a metal salt of the peptide derivative represented by Formula (I), wherein the metal includes lithium, sodium, potassium, calcium, magnesium, manganese, copper, zinc or aluminum;
optionally, the cosmetically acceptable salt or pharmaceutically acceptable salt includes a salt formed from the peptide derivative represented by Formula (I) and an organic alkali, wherein the organic alkali includes ethylamine, diethylamine, ethylenediamine, ethanolamine, diethanolamine, arginine, lysine, histidine or piperazine;
optionally, the cosmetically acceptable salt or pharmaceutically acceptable salt includes a salt formed from the peptide derivative represented by Formula (I) and an inorganic acid or an organic acid, wherein the organic acid includes acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid;
optionally, the inorganic acid includes hydrochloric acid, sulfuric acid, boric acid or carbonic acid.

5. A cosmetic or pharmaceutical composition, **characterized in that** it includes an effective amount of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, and at least one excipient and optionally a cosmetically or pharmaceutically acceptable adjuvant;
optionally, the adjuvant is selected from a collagen synthesis stimulator, an agent that modulates the PGC-1α synthesis, an agent that modulates the activity of PPARγ, an agent that increases or decreases a triglyceride content in adipocytes, an agent that stimulates or delays adipocyte differentiation, a lipolytic agent or an agent that simulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinases inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates the HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, an comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, an agents derived from a biological fermentation process, an inorganic salt, a cell extract, a sunscreen, and an effective organic or inorganic photoprotectant against A and/or B UV rays, or mixtures thereof.

6. The cosmetic or pharmaceutical composition according to claim 5, **characterized in that** a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or gelatin;
optionally, the capsule includes a soft capsule, a hard capsule, optionally a gelatin capsule;
optionally, the tablet includes a sugar-coated tablet.

7. A cosmetically or pharmaceutically acceptable delivery system or sustained release system, **characterized in that** it comprises an effective amount of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, or the cosmetic or pharmaceutical composition according to claim 5 or 6;
the cosmetically or pharmaceutically acceptable delivery system or sustained release system is selected from a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle; or optionally a liposome or microemulsion; or optionally a water-in-oil microemulsion having an internal structure of reverse micelle.

8. Use of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, or the cosmetic or pharmaceutical composition according to claim 5 or 6, or the cosmetically or pharmaceutically acceptable delivery system or sustained release system according to claim 7 in the preparation of a cosmetic composition or pharmaceutical composition for treatment or care of skin or mucosa.

9. Use of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, or the cosmetic or pharmaceutical composition according to claim 5 or 6, or the cosmetically or pharmaceutically acceptable delivery system or sustained release system according to claim 7 in the preparation of a cosmetic composition or pharmaceutical composition for whitening.

10. Use of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, or the cosmetic or pharmaceutical composition according to claim 5 or 6, or the cosmetically or pharmaceutically acceptable delivery system or sustained release system according to claim 7 in the preparation of a cosmetic composition or pharmaceutical composition for anti-inflammation.

11. Use of the peptide derivative represented by Formula (I) according to any of claims 1-4, or a stereoisomer thereof, or a mixture of stereoisomers thereof, or a cosmetically acceptable salt thereof, or a pharmaceutically acceptable salt thereof, or the cosmetic or pharmaceutical composition according to claim 5 or 6, or the cosmetically or pharmaceutically acceptable delivery system or sustained release system according to claim 7 in the preparation of a cosmetic composition or pharmaceutical composition for improving post-inflammation hyperpigmentation.

## Patentansprüche

1. Ein Peptidderivat, dargestellt durch Formel (I), oder ein Stereoisomer davon oder ein Gemisch von Stereoisomeren davon oder ein kosmetisch verträgliches Salz davon oder ein pharmazeutisch verträgliches Salz davon,
R₁-Pro-Lys-Glu-Lys-R₂ (I)
in Formel (I),
R₁ ist ausgewählt aus dem Substituent (i) oder Substituent (ii), wobei Substituent (i) ist 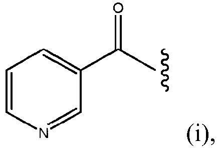
wobei der Substituent (ii) ist 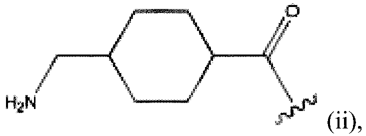
R₂ ist ausgewählt aus -NR₃R₄ oder -OR₃, wobei R₃ und R₄ jeweils unabhängig ausgewählt sind aus H, substituiertem oder unsubstituiertem Alkyl, substituiertem oder unsubstituiertem Alkenyl;
das Alkyl bezieht sich auf ein gesättigtes aliphatisches, lineares oder verzweigtes Alkyl mit 1-24 Kohlenstoffatomen (optional mit 1-16 Kohlenstoffatomen; optional mit 1-14 Kohlenstoffatomen; optional mit 1-12 Kohlenstoffatomen; oder optional mit 1, 2, 3, 4, 5 oder 6 Kohlenstoffatomen); wobei das Alkyl optional ausgewählt ist aus Methyl, Ethyl, Isopropyl, Isobutyl, *tert*-Butyl, Pentyl, Hexyl, Heptyl, Octyl, Decyl, Dodecyl, Tetradecyl, Hexadecyl, Octadecyl, 2-Ethylhexyl, 2-Methylbutyl oder 5-Methylhexyl;
das Alkenyl bezieht sich auf ein lineares oder verzweigtes Alkenyl mit 2-24 Kohlenstoffatomen (optional mit 2-16 Kohlenstoffatomen; optional mit 2-14 Kohlenstoffatomen; optional mit 2-12 Kohlenstoffatomen; optional mit 2, 3, 4, 5 oder 6 Kohlenstoffatomen); das Alkenyl weist eine oder mehrere Kohlenstoff-Kohlenstoff-Doppelbindungen auf oder optional 1, 2 oder 3 konjugierte oder nicht konjugierte Kohlenstoff-Kohlenstoff-Doppelbindungen; das Alkenyl ist über eine Einfachbindung mit den übrigen Anteilen des Moleküls verbunden, optional ausgewählt aus Vinyl, Oleyl oder Linoleyl;
optional ist der Substituent im "substituted alkyl" oder "substituted alkenyl" ausgewählt aus C₁-C₄-Alkyl; Hydroxyl; C₁-C₄-Alkoxy; Amino; C₁-C₄-Aminoalkyl; C₁-C₄-Carbonyloxy; C₁-C₄-Oxycarbonyl; Halogen (wie Fluor, Chlor, Brom und Iod); Cyano; Nitro; Azid; C₁-C₄-Alkylsulfonyl; Thiol; C₁-C₄-Alkylthio; C₆-C₃₀-Aryloxy wie Phenoxy; -NR_{b}(C=NR_{b}) NR_{b}R_{c}, wobei R_{b} und R_{c} jeweils unabhängig ausgewählt sind aus H, C₁-C₄-Alkyl, C₂-C₄₋Alkenyl, C₂₋C₄-Alkynyl, C₃-C₁₀-Cycloalkyl, C₆-C₁₈-Aryl, C₇-C₁₇-Aralkyl, einer heterocyclischen Gruppe mit 3 bis 10 Gliedern oder einer Aminschutzgruppe.

2. Das Peptidderivat, dargestellt durch Formel (I) nach Anspruch 1, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass** R₃ und R₄ jeweils unabhängig ausgewählt sind aus H, Methyl, Ethyl, Hexyl, Dodecyl oder Hexadecyl;
optional ist R₃ H und R₄ ist ausgewählt aus H, Methyl, Ethyl, Hexyl, Dodecyl oder Hexadecyl;
optional ist R₂ -OH oder -NH₂.

3. Das Peptidderivat, dargestellt durch Formel (I) nach einem der Ansprüche 1-2, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass** es ausgewählt ist aus den folgenden Peptidderivaten (1)-(4):

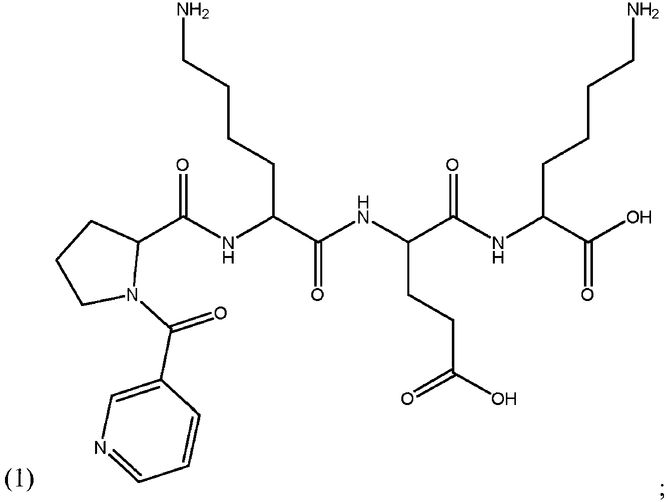

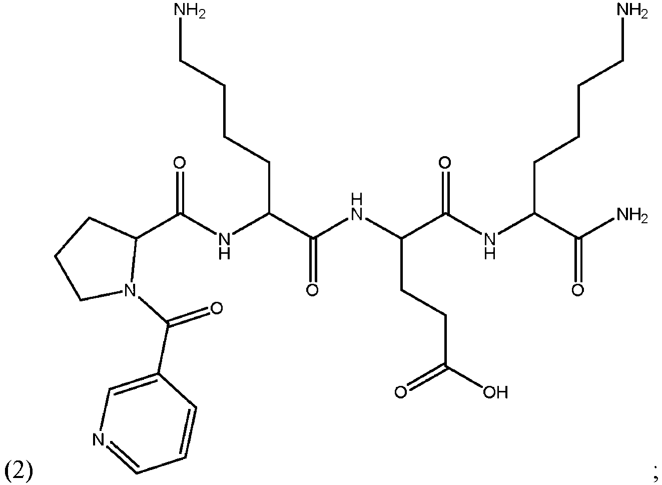

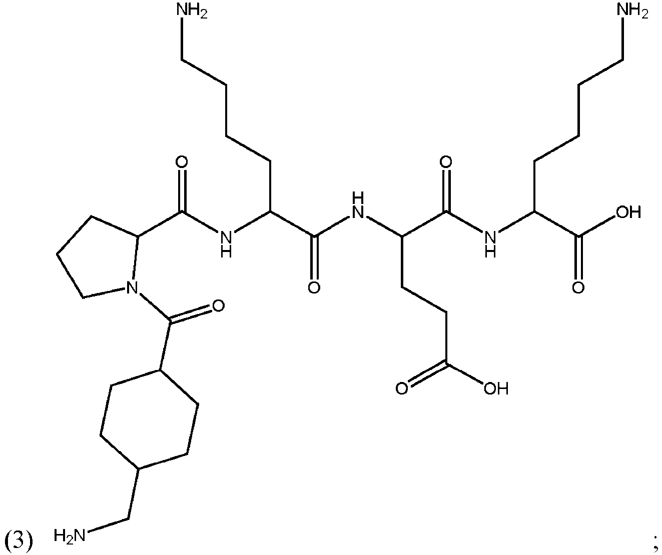

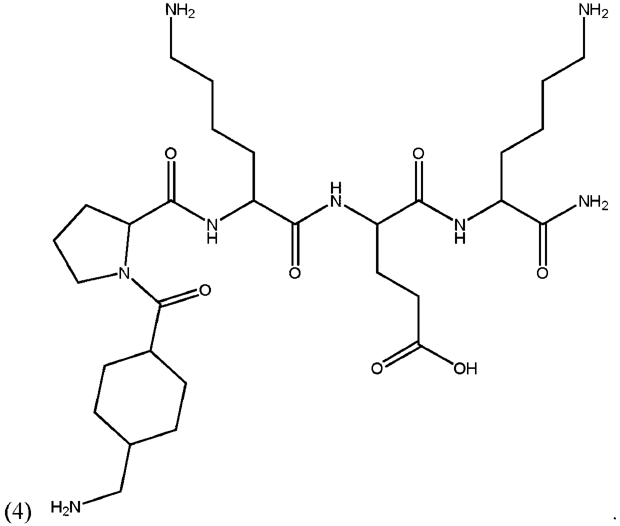

4. Das Peptidderivat, dargestellt durch Formel (I) nach Anspruch 1, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass**
das kosmetisch verträgliche oder pharmazeutisch verträgliche Salz ein Metallsalz des durch Formel (I) dargestellten Peptidderivats umfasst, wobei das Metall Lithium, Natrium, Kalium, Calcium, Magnesium, Mangan, Kupfer, Zink oder Aluminium umfasst;
optional umfasst das kosmetisch verträgliche oder pharmazeutisch verträgliche Salz ein Salz, gebildet aus dem durch Formel (I) dargestellten Peptidderivat und einer organischen Base, wobei die organische Base Ethylamin, Diethylamin, Ethylendiamin, Ethanolamin, Diethanolamin, Arginin, Lysin, Histidin oder Piperazin umfasst;
optional umfasst das kosmetisch verträgliche oder pharmazeutisch verträgliche Salz ein Salz, gebildet aus dem durch Formel (I) dargestellten Peptidderivat und einer anorganischen oder organischen Säure, wobei die organische Säure Essigsäure, Citronensäure, Milchsäure, Malonsäure, Maleinsäure, Weinsäure, Fumarsäure, Benzoesäure, Asparaginsäure, Glutaminsäure, Bernsteinsäure, Ölsäure, Trifluoressigsäure, Oxalsäure, Pamoesäure oder Gluconsäure umfasst;
optional umfasst die anorganische Säure Salzsäure, Schwefelsäure, Borsäure oder Kohlensäure.

5. Eine kosmetische oder pharmazeutische Zusammensetzung, **dadurch gekennzeichnet, dass** sie eine wirksame Menge des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon umfasst und mindestens ein Hilfsmittel und optional einen kosmetisch oder pharmazeutisch verträglichen Zusatzstoff;
optional ist der Zusatzstoff ausgewählt aus einem Kollagensynthesestimulator, einem Mittel zur Modulation der PGC-1α-Synthese, einem Mittel zur Modulation der Aktivität von PPARγ, einem Mittel zur Erhöhung oder Verringerung eines Triglyceridgehalts in Adipozyten, einem Mittel zur Stimulierung oder Verzögerung der Adipozytendifferenzierung, einem lipolytischen Mittel oder einem Mittel zur Stimulation der Lipolyse, einem Fettklärmittel, einem adipogenen Mittel, einem Inhibitor gegen Acetylcholinrezeptoraggregation, einem Mittel zur Hemmung der Muskelkontraktion, einem anticholinergen Mittel, einem Elastaseinhibitor, einem Matrix-Metalloproteinase-Inhibitor, einem Melaninsynthesestimulator oder -inhibitor, einem Weißmacher oder Entfärbungsmittel, einem Pigmentierungsförderer, einem Selbstbräuner, einem Anti-Aging-Mittel, einem NO-Synthase-Inhibitor, einem 5α-Reduktase-Inhibitor, einem Inhibitor gegen Lysylhydroxylase und/oder Prolylhydroxylase, einem Antioxidans, einem Radikalfänger und/oder einem Anti-Luftverschmutzungs-Mittel, einem Reaktiv-Carbonyl-Spezies-Fänger, einem Anti-Glykations-Mittel, einem Antihistaminikum, einem Virustatikum, einem Antiparasitikum, einem Emulgator, einem Emolliens, einem organischen Lösungsmittel, einem flüssigen Treibmittel, einem Hautkonditionierer, einem Feuchthaltemittel, einer Feuchtigkeitsbewahrungssubstanz, einer Alpha-Hydroxy-Säure, einer Beta-Hydroxy-Säure, einem Feuchtigkeitsspender, einer epidermalen Hydrolase, einem Vitamin, einer Aminosäure, einem Protein, einem Pigment oder Färbemittel, einem Farbstoff, einem Biopolymer, einem Gelierpolymer, einem Verdickungsmittel, einem Tensid, einem Weichmacher, einem Haftmittel, einem Konservierungsmittel, einem Anti-Falten-Mittel, einem Mittel zur Reduzierung oder Behandlung von Unter-Augen-Schwellungen, einem Exfoliant, einem Mittel zur Cuticulenentfernung, einem keratolytischen Mittel, einem antimikrobiellen Mittel, einem Antimykotikum, einem Fungistatikum, einem Bakterizid, einem Bakteriostatikum, einem Mittel zur Stimulierung der Synthese dermaler oder epidermaler Makromoleküle und/oder zur Hemmung oder Verhinderung deren Abbaus, einem Mittel zur Stimulierung der Elastinsynthese, einem Mittel zur Stimulierung der Decorinsynthese, einem Mittel zur Stimulierung der Lamininsynthese, einem Mittel zur Stimulierung der Defensinsynthese, einem Mittel zur Stimulierung der Chaperon-Proteinsynthese, einem Mittel zur Stimulierung der cAMP-Synthese, einem Hitzeschockprotein, einem Mittel zur Stimulierung der HSP70-Synthese, einem Mittel zur Stimulierung der Synthese eines Hitzeschockproteins, einem Mittel zur Stimulierung der Synthese von Hyaluronsäure, einem Mittel zur Stimulierung der Fibronectinsynthese, einem Mittel zur Stimulierung der Deacetylasesynthese, einem Mittel zur Stimulierung der Synthese eines Lipids und einer Stratum-corneum-Komponente, Ceramid, einer Fettsäure, einem Mittel zur Hemmung des Kollagenabbaus, einem Mittel zur Hemmung des Elastinabbaus, einem Mittel zur Hemmung der Serinprotease, einem Mittel zur Stimulierung der Fibroblastenproliferation, einem Mittel zur Stimulierung der Keratinozytenproliferation, einem Mittel zur Stimulierung der Adipozytenproliferation, einem Mittel zur Stimulierung der Melanozytenproliferation, einem Mittel zur Stimulierung der Keratinozytendifferenzierung, einem Mittel zur Hemmung der Acetylcholinesterase, einem Hautrelaxans, einem Mittel zur Stimulierung der Glykosaminoglykansynthese, einem Anti-Hyperkeratose-Mittel, einem komedolytischen Mittel, einem Antipsoriatikum, einem Anti-Dermatitis-Mittel, einem Anti-Ekzema-Mittel, einem DNA-Reparaturmittel, einem DNA-Schutzmittel, einem Stabilisator, einem Antipruriginosum, einem Mittel zur Behandlung und/oder Pflege empfindlicher Haut, einem Härtungsmittel, einem Straffungsmittel, einem Restrukturierungsmittel, einem Anti-Stretching-Mittel, einem Haftmittel, einem Mittel zur Modulation der Talgproduktion, einem Antitranspirant, einem Mittel zur Stimulierung der Heilung, einem Mittel zur Unterstützung der Heilung, einem Mittel zur Stimulierung der Re-Epithelisierung, einem Mittel zur Unterstützung der Re-Epithelisierung, einem Zytokin-Wachstumsfaktor, einem Sedativum, einem Antiphlogistikum, einem Anästhetikum, einem Mittel mit Wirkung auf die Kapillarzirkulation und/oder Mikrozirkulation, einem Mittel zur Stimulierung der Angiogenese, einem Mittel zur Hemmung der Gefäßpermeabilität, einem Venotonikum, einem Mittel mit Wirkung auf den Zellstoffwechsel, einem Mittel zur Verbesserung der Dermis-Epidermis-Verbindung, einem Mittel zur Induktion des Haarwuchses, einem Mittel zur Hemmung oder Verzögerung des Haarwuchses, einem Duftstoff, einem Chelatbildner, einem Pflanzenextrakt, einem ätherischen Öl, einem Meeresextrakt, Mitteln, die aus einem biologischen Fermentationsprozess gewonnen sind, einem anorganischen Salz, einem Zellextrakt, einem Sonnenschutzmittel und einem wirksamen organischen oder anorganischen Photoprotektivum gegen A- und/oder B-UV-Strahlung oder Mischungen davon.

6. Die kosmetische oder pharmazeutische Zusammensetzung nach Anspruch 5, **dadurch gekennzeichnet, dass** eine Zubereitung der kosmetischen oder pharmazeutischen Zusammensetzung ausgewählt ist aus einer Creme, einem Öl, einer Milch, einem Balsam, einem Schaum, einer Lotion, einem Gel, einem Liniment, einem Serum, einer Seife, einem Shampoo, einem Haarconditioner, einer Salbe, einem Mousse, einer Pomade, einem Puder, einem Riegel, einem Stift, einem Spray, einem Aerosol, einer Kapsel, einer Tablette, einem Granulat, einem Kaugummi, einer Lösung, einer Suspension, einer Emulsion, einem Sirup, einem Elixier, einem Polysaccharidfilm, einem Gelfilm oder Gelatine;
optional umfasst die Kapsel eine Weichkapsel, eine Hartkapsel, optional eine Gelatinekapsel;
optional umfasst die Tablette eine Drageetablette.

7. Ein kosmetisch oder pharmazeutisch verträgliches Abgabesystem oder Wirkstofffreisetzungssystem, **dadurch gekennzeichnet, dass** es eine wirksame Menge des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon oder der kosmetischen oder pharmazeutischen Zusammensetzung nach Anspruch 5 oder 6 umfasst;
das kosmetisch oder pharmazeutisch verträgliche Abgabesystem oder Wirkstofffreisetzungssystem ist ausgewählt aus einem Liposom, einem Oleosom, einem nichtionischen Tensid-Liposomenvesikel, einem Ethosom, einer Millicapsel, einer Mikrokapsel, einer Nanokapsel, einem nanostrukturierten Lipidträger, einem Schwamm, einem Cyclodextrin, einem lipoiden Vesikel, einer Mizelle, einer Millisphäre, einer Mikrosphäre, einer Nanosphäre, einer Liposphäre, einer Mikroemulsion, einer Nanoemulsion, einem Millipartikel, einem Mikropartikel oder einem Nanopartikel; oder optional einem Liposom oder einer Mikroemulsion; oder optional einer Wasser-in-Öl-Mikroemulsion mit einer inneren Struktur aus inversen Mizellen.

8. Verwendung des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon oder der kosmetischen oder pharmazeutischen Zusammensetzung nach Anspruch 5 oder 6 oder des kosmetisch oder pharmazeutisch verträglichen Abgabesystems oder Wirkstofffreisetzungssystems nach Anspruch 7 bei der Herstellung einer kosmetischen Zusammensetzung oder pharmazeutischen Zusammensetzung zur Behandlung oder Pflege der Haut oder Schleimhaut.

9. Verwendung des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon oder der kosmetischen oder pharmazeutischen Zusammensetzung nach Anspruch 5 oder 6 oder des kosmetisch oder pharmazeutisch verträglichen Abgabesystems oder Wirkstofffreisetzungssystems nach Anspruch 7 bei der Herstellung einer kosmetischen Zusammensetzung oder pharmazeutischen Zusammensetzung zur Hautaufhellung.

10. Verwendung des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon oder der kosmetischen oder pharmazeutischen Zusammensetzung nach Anspruch 5 oder 6 oder des kosmetisch oder pharmazeutisch verträglichen Abgabesystems oder Wirkstofffreisetzungssystems nach Anspruch 7 bei der Herstellung einer kosmetischen Zusammensetzung oder pharmazeutischen Zusammensetzung zur Entzündungshemmung.

11. Verwendung des durch Formel (I) dargestellten Peptidderivats nach einem der Ansprüche 1-4 oder eines Stereoisomers davon oder eines Gemischs von Stereoisomeren davon oder eines kosmetisch verträglichen Salzes davon oder eines pharmazeutisch verträglichen Salzes davon oder der kosmetischen oder pharmazeutischen Zusammensetzung nach Anspruch 5 oder 6 oder des kosmetisch oder pharmazeutisch verträglichen Abgabesystems oder Wirkstofffreisetzungssystems nach Anspruch 7 bei der Herstellung einer kosmetischen Zusammensetzung oder pharmazeutischen Zusammensetzung zur Verbesserung einer postinflammatorischen Hyperpigmentierung.

## Revendications

1. Dérivé peptidique représenté par la Formule (I), ou un stéréoisomère de celui-ci, ou un mélange de stéréoisomères de celui-ci, ou un sel cosmétiquement acceptable de celui-ci, ou un sel pharmaceutiquement acceptable de celui-ci,
R₁-Pro-Lys-Glu-Lys-R₂ (I)
dans la Formule (I),
R₁ est choisi parmi le substituant (i) ou le substituant (ii), dans lequel le substituant (i) est 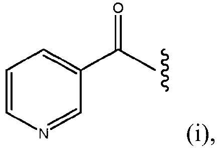
le substituant (ii) est 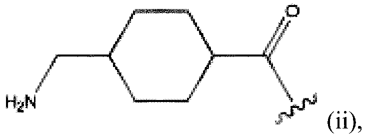
R₂ est choisi parmi un groupe -NR₃R₄ ou un groupe -OR₃, où chaque R₃ et R₄ est indépendamment choisi parmi un atome H, un groupe alkyle substitué ou non-substitué, un groupe alcényle substitué ou non-substitué ;
le groupe alkyle désigne une chaîne alkyle linéaire ou ramifiée aliphatique saturée ayant 1-24 atomes de carbone (ayant optionnellement 1-16 atomes de carbone ; ayant optionnellement 1-14 atomes de carbone ; ayant optionnellement 1-12 atomes de carbone ; ou ayant optionnellement 1, 2, 3, 4, 5 ou 6 atomes de carbone) ; optionnellement est choisi parmi un groupe méthyle, un groupe éthyle, un groupe isopropyle, un groupe isobutyle, un groupe *tert*-butyle, un groupe pentyle, un groupe hexyle, un groupe heptyle, un groupe octyle, un groupe décyle, un groupe dodécyle, un groupe tétradécyle, un groupe hexadécyle, un groupe octadécyle, un groupe 2-éthylhexyle, un groupe 2-méthylbutyle, ou un groupe 5-méthylhexyle ;
le groupe alcényle désigne une chaîne alcényle linéaire ou ramifiée ayant 2-24 atomes de carbone (ayant éventuellement 2-16 atomes de carbone ; ayant optionnellement 2-14 atomes de carbone ; ayant optionnellement 2-12 atomes de carbone ; ayant optionnellement 2, 3, 4, 5 ou 6 atomes de carbone) ; le groupe alcényle présente une ou plusieurs liaisons doubles carbone-carbone, ou présente optionnellement 1, 2 ou 3 liaisons doubles carbone-carbone conjuguées ou non-conjuguées ; le groupe alcényle est lié aux parties de repos d'une molécule à travers une liaison simple, optionnellement est choisi parmi un groupe vinyle, un groupe oléyle ou un groupe linoléyle ;
optionnellement, le substituant dans « l'alkyle substitué » ou « l'alcényle substitué » est choisi parmi un groupe alkyle en C₁-C₄ ; un groupe hydroxyle ; un groupe alcoxy en C₁-C₄ ; un groupe amino ; un groupe aminoalkyle en C₁-C₄ ; un groupe carbonyloxy en C₁-C₄ ; un groupe oxycarbonyle en C₁-C₄ ; un halogène (tel que le fluor, le chlore, le brome et l'iode) ; un groupe cyano ; un groupe nitro ; un groupe azoture ; un groupe alkylsulfonyle en C₁-C₄ ; un groupe thiol ; un groupe alkylthio en C₁-C₄ ; un groupe aryloxy en C₆-C₃₀ tel qu'un groupe phénoxy ; un groupe -NR₆(C=NR_{b})NR_{b}R_{c}, où R_{b} et R_{c} sont indépendamment choisis parmi H, un groupe alkyle en C₁-C₄, un groupe alcényle en C₂-C₄, un groupe alcynyle en C₂-C₄, un groupe cycloalkyle en C₃-C₁₀, un groupe aryle en C₆-C₁₈, un groupe aralkyle en C₇-C₁₇, un groupe hétérocyclique ayant 3 à 10 chaînons, ou un groupe amino-protecteur.

2. Dérivé peptidique représenté par la Formule (I) selon la revendication 1, ou un stéréoisomère de celui-ci, ou un mélange de stéréoisomères de celui-ci, ou un sel cosmétiquement acceptable de celui-ci, ou un sel pharmaceutiquement acceptable de celui-ci, **caractérisé en ce que** R₃ et R₄ sont indépendamment choisis parmi un atome H, un groupe méthyle, un groupe éthyle, un groupe hexyle, un groupe dodécyle ou un groupe hexadécyle ;
optionnellement, R₃ est un atome H et R₄ est choisi parmi un atome H, un groupe méthyle, un groupe éthyle, un groupe hexyle, un groupe dodécyle ou un groupe hexadécyle ;
optionnellement, R₂ est un groupe -OH ou un groupe -NH₂.

3. Dérivé peptidique représenté par la Formule (I) selon l'une quelconque des revendications 1-2, ou un stéréoisomère de celui-ci, ou un mélange de stéréoisomères de celui-ci, ou un sel cosmétiquement acceptable de celui-ci, ou un sel pharmaceutiquement acceptable de celui-ci, **caractérisé en ce qu'**il est choisi parmi les dérivés peptidiques suivants (1)-(4) :

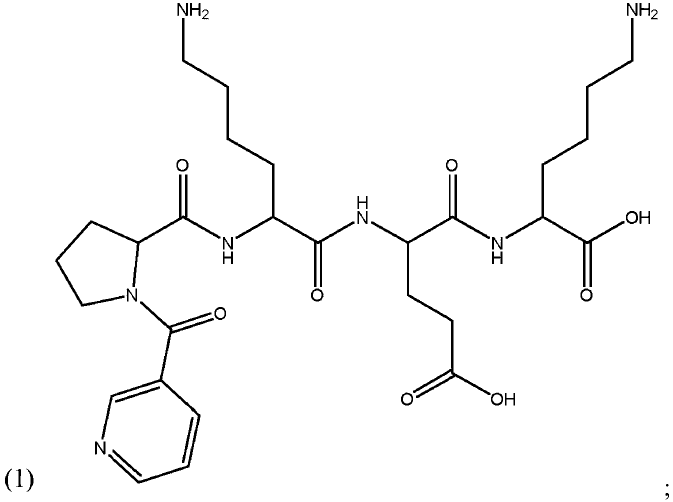

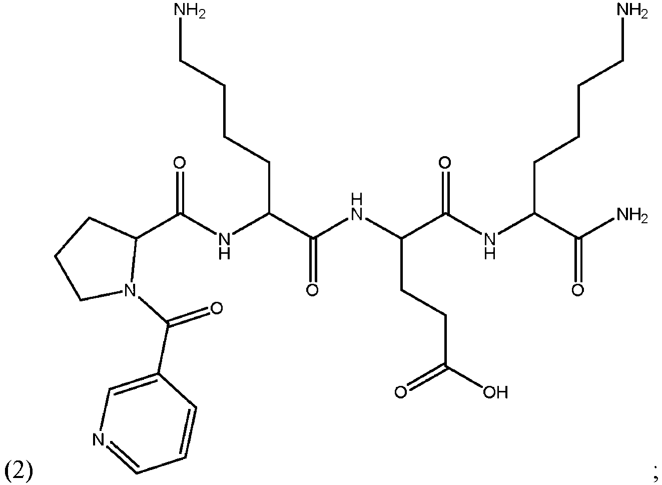

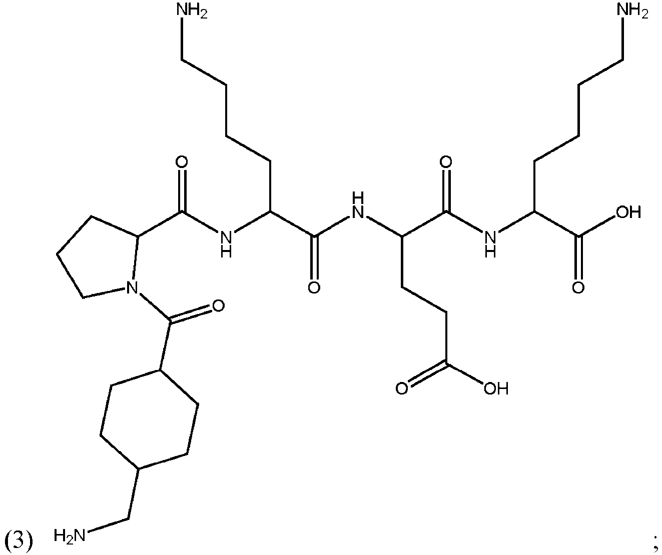

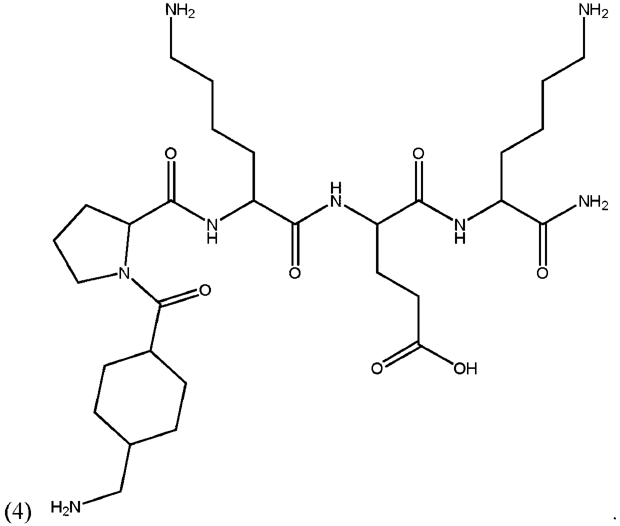

4. Dérivé peptidique représenté par la Formule (I) selon la revendication 1, ou un stéréoisomère de celui-ci, ou un mélange de stéréoisomères de celui-ci, ou un sel cosmétiquement acceptable de celui-ci, ou un sel pharmaceutiquement acceptable de celui-ci, **caractérisé en ce que**
le sel cosmétiquement acceptable ou le sel pharmaceutiquement acceptable comporte un sel métallique du dérivé peptidique représenté par la Formule (I), dans lequel le métal comporte du lithium, du sodium, du potassium, du calcium, du magnésium, du manganèse, du cuivre, du zinc ou de l'aluminium ;
optionnellement, le sel cosmétiquement acceptable ou le sel pharmaceutiquement acceptable comporte un sel formé à partir du dérivé peptidique représenté par la Formule (I) et une base organique, dans lequel la base organique comporte de l'éthylamine, de la diéthylamine, de l'éthylènediamine, de l'éthanolamine, de la diéthanolamine, de l'arginine, de la lysine, de l'histidine ou de la pipérazine ;
optionnellement, le sel cosmétiquement acceptable ou le sel pharmaceutiquement acceptable comporte un sel formé à partir du dérivé peptidique représenté par la Formule (I) et un acide inorganique ou un acide organique, dans lequel l'acide organique comporte de l'acide acétique, de l'acide citrique, de l'acide lactique, de l'acide malonique, de l'acide maléique, de l'acide tartrique, de l'acide fumarique, de l'acide benzoïque, de l'acide aspartique, de l'acide glutamique, de l'acide succinique, de l'acide oléique, de l'acide trifluoroacétique, de l'acide oxalique, de l'acide pamoïque, ou de l'acide gluconique ;
optionnellement, l'acide inorganique comporte de l'acide chlorhydrique, de l'acide sulfurique, de l'acide borique ou de l'acide carbonique.

5. Composition cosmétique ou pharmaceutique, **caractérisée en ce qu'**elle comporte une quantité effective du dérivé peptidique représenté par la Formule (I) selon l'une des revendications 1-4, ou un stéréoisomère de celui-ci, ou un mélange de stéréoisomères de celui-ci, ou un sel cosmétiquement acceptable de celui-ci, ou un sel pharmaceutiquement acceptable de celui-ci, et au moins un excipient et optionnellement un adjuvant cosmétiquement ou pharmaceutiquement acceptable ;
optionnellement, l'adjuvant est choisi parmi un stimulateur de synthèse de collagène, un agent qui module la synthèse de PGC-1α, un agent qui module l'activité de PPARγ, un agent qui augmente ou diminue une teneur en triglycérides dans les adipocytes, un agent qui stimule ou retarde la différenciation adipocytaire, un agent lipolytique ou un agent qui stimule la lipolyse, un agent de compensation de graisse, un agent adipogénique, un inhibiteur contre l'agrégation de récepteurs d'acétylcholine, un agent qui inhibe la contraction musculaire, un agent anticholinergique, un inhibiteur d'élastase, un inhibiteur de métalloprotéinases matricielles, un stimulateur ou inhibiteur de synthèse de mélanine, un agent blanchissant ou décolorant, un promoteur de pigmentation, un agent autobronzant, un agent antivieillissement, un inhibiteur de NO-synthase, un inhibiteur de 5α-reductase, un inhibiteur contre la lysyl hydroxylase et/ou la prolyl hydroxylase, un antioxydant, un piégeur de radicaux libres et/ou un agent contre la pollution de l'air, un piégeur d'espèces carbonyle réactives, un agent anti-glycation, un antihistaminique, un agent antiviral, un agent antiparasitaire, un émulsifiant, un émollient, un solvant organique, un propulseur liquide, un conditionneur de peau, un humectant, une substance retenant l'humidité, un acide alpha-hydroxylé, un acide beta-hydroxylé, un hydratant, une hydrolase épidermique, une vitamine, un acide aminé, une protéine, un pigment ou un colorant, une teinture, un biopolymère, un polymère gélifiant, un épaississant, un tensioactif, un adoucissant, un adhésif, un conservateur, un agent antirides, un agent qui peut réduire ou traiter des poches sous-oculaires, un exfoliant, un agent de décapage de cuticule, un agent kératolytique, un antimicrobien, un antifongique, un fongistat, un bactéricide, un bactériostat, un agent qui stimule la synthèse de macromolécules dermiques ou épidermiques et/ou inhibe ou empêche leur dégradation, un agent qui stimule la synthèse d'élastine, un agent qui stimule la synthèse de décorine, un agent qui stimule la synthèse de laminine, un agent qui stimule la synthèse de défensine, un agent qui stimule la synthèse de protéine chaperon, un agent qui stimule la synthèse d'AMPc, une protéine de choc thermique, un agent qui stimule la synthèse de HSP70, un agent qui stimule la synthèse d'une protéine de choc thermique, un agent qui stimule la synthèse d'acide hyaluronique, un agent qui stimule la synthèse de la fibronectine, un agent qui stimule la synthèse de désacétylase, un agent qui stimule la synthèse d'un lipide et d'un composant de stratum comeum, un céramide, un acide gras, un agent qui inhibe la dégradation du collagène, un agent qui inhibe la dégradation de l'élastine, un agent qui inhibe la sérine protéase, un agent qui stimule la prolifération des fibroblastes, un agent qui stimule la prolifération des kératinocytes, un agent qui stimule la prolifération des adipocytes, un agent qui stimule la prolifération des mélanocytes, un agent qui stimule la différenciation des kératinocytes, un agent qui inhibe l'acétylcholinestérase, un relaxant de la peau, un agent qui stimule la synthèse de glycosaminoglycane, un agent anti-hyperkératose, un agent comédolytique, un agent antipsoriasique, un agent anti-dermatite, un agent anti-eczéma, un agent de réparation de l'ADN, un agent de protection de l'ADN, un stabilisant, un agent antiprurigineux, un agent pour le traitement et/ou le soin de la peau sensible, un agent de durcissement, un agent de raidissement, un agent de restructuration, un agent anti-étirement, un adhésif, un agent qui module la production de sébum, un antisudorifique, un agent qui stimule la cicatrisation, un agent qui assiste la cicatrisation, un agent qui stimule la re-épithélialisation, un agent qui assiste la re-épithélialisation, un facteur de croissance des cytokines, un sédatif, un agent anti-inflammatoire, un agent anesthésique, un agent agissant sur la circulation capillaire et/ou la microcirculation, un agent qui stimule l'angiogenèse, un agent qui inhibe la perméabilité vasculaire, un agent veinotonique, un agent agissant sur le métabolisme cellulaire, un agent qui améliore la liaison derme-épiderme, un agent qui induit la pousse des cheveux, un agent inhibiteur ou retardateur de croissance capillaire, un parfum, un agent chélatant, un extrait végétal, une huile essentielle, un extrait marin, un agent dérivé d'un procédé de fermentation biologique, un sel inorganique, un extrait cellulaire, un écran solaire, et un photo-protecteur organique ou inorganique efficace contre les rayons UV A et/ou B, ou des mélanges de ceux-ci.

6. Composition cosmétique ou pharmaceutique selon la revendication 5, **caractérisée en ce qu'**une préparation de la composition cosmétique ou pharmaceutique est choisie parmi une crème, une huile, un lait, un baume, une mousse, une lotion, un gel, un liniment, un sérum, un savon, un shampooing, un après-shampooing, un sérum, un onguent, une mousse coiffante, une pommade, une poudre, une barre, un crayon, un spray, un aérosol, une capsule, un comprimé, un granulé, un chewing-gum, une solution, une suspension, une émulsion, un sirop, un élixir, un film de polysaccharide, une gelée ou de la gélatine ;
optionnellement, la capsule comporte une capsule molle, une capsule dure, optionnellement une capsule de gélatine ;
optionnellement, le comprimé comporte un comprimé enrobé de sucre.

7. Système d'administration cosmétiquement ou pharmaceutiquement acceptable ou système de libération prolongée, **caractérisé en ce qu'**il comprend une quantité effective du dérivé peptidique représenté par la Formule (I) selon l'une des revendications 1-4, ou un stéréoisomère de celui-ci, ou un mélange de stéréoisomères de celui-ci, ou un sel cosmétiquement acceptable de celui-ci, ou un sel pharmaceutiquement acceptable de celui-ci, ou la composition cosmétique ou pharmaceutique selon la revendication 5 ou 6 ;
le système d'administration cosmétiquement ou pharmaceutiquement acceptable ou le système de libération prolongée est choisi parmi un liposome, une oléosome, une vésicule de liposome de tensioactif non ionique, un éthosome, une millicapsule, une microcapsule, une nanocapsule, un support lipidique nanostructuré, une éponge, une cyclodextrine, une vésicule de lipoïde, une micelle, une millisphère, une microsphère, une nanosphère, une liposphère, une microémulsion, une nanoémulsion, une milliparticule, une microparticule ou une nanoparticule ; ou optionnellement un liposome ou une microémulsion ; ou optionnellement une microémulsion de type eau dans l'huile présentant une structure interne de micelle inverse.

8. Utilisation du dérivé peptidique représenté par la Formule (I) selon l'une des revendications 1-4, ou d'un stéréoisomère de celui-ci, ou d'un mélange de stéréoisomères de celui-ci, ou d'un sel cosmétiquement acceptable de celui-ci, ou d'un sel pharmaceutiquement acceptable de celui-ci, ou de la composition cosmétique ou pharmaceutique selon la revendication 5 ou 6, ou du système d'administration cosmétiquement ou pharmaceutiquement acceptable ou du système de libération prolongée selon la revendication 7 dans la préparation d'une composition cosmétique ou d'une composition pharmaceutique pour le traitement ou le soin de la peau ou des muqueuses.

9. Utilisation du dérivé peptidique représenté par la Formule (I) selon l'une des revendications 1-4, ou d'un stéréoisomère de celui-ci, ou d'un mélange de stéréoisomères de celui-ci, ou d'un sel cosmétiquement acceptable de celui-ci, ou d'un sel pharmaceutiquement acceptable de celui-ci, ou de la composition cosmétique ou pharmaceutique selon la revendication 5 ou 6, ou du système d'administration cosmétiquement ou pharmaceutiquement acceptable ou du système de libération prolongée selon la revendication 7 dans la préparation d'une composition cosmétique blanchissante ou d'une composition pharmaceutique blanchissante.

10. Utilisation du dérivé peptidique représenté par la Formule (I) selon l'une des revendications 1-4, ou d'un stéréoisomère de celui-ci, ou d'un mélange de stéréoisomères de celui-ci, ou d'un sel cosmétiquement acceptable de celui-ci, ou d'un sel pharmaceutiquement acceptable de celui-ci, ou de la composition cosmétique ou pharmaceutique selon la revendication 5 ou 6, ou du système d'administration cosmétiquement ou pharmaceutiquement acceptable ou du système de libération prolongée selon la revendication 7 dans la préparation d'une composition cosmétique anti-inflammatoire ou d'une composition pharmaceutique anti-inflammatoire.

11. Utilisation du dérivé peptidique représenté par la Formule (I) selon l'une des revendications 1-4, ou d'un stéréoisomère de celui-ci, ou d'un mélange de stéréoisomères de celui-ci, ou d'un sel cosmétiquement acceptable de celui-ci, ou d'un sel pharmaceutiquement acceptable de celui-ci, ou de la composition cosmétique ou pharmaceutique selon la revendication 5 ou 6, ou du système d'administration cosmétiquement ou pharmaceutiquement acceptable ou du système de libération prolongée selon la revendication 7 dans la préparation d'une composition cosmétique ou d'une composition pharmaceutique pour l'amélioration de l'hyperpigmentation post-inflammation.
